# EUROPEAN PATENT SPECIFICATION

(11) **EP 1 654 360 B1**
(45) Date of publication and mention of the grant of the patent: **21.11.2007**
(21) Application number: 04743697.7
(22) Date of filing: 12.08.2004
(51) Int. Cl.: C12N 15/10, C12P 19/34, C12Q 1/68

(54) **AMPLIFICATION METHOD**
AMPLIFIKATIONSMETHODE
PROCEDE D'AMPLIFICATION

(30) Priority: 16.08.2003 GB 0319332
(43) Date of publication of application: 10.05.2006
(73) Proprietor: AstraZeneca AB, 151 85 (SE)
(72) Inventor: GRAHAM, Alexander, Macclesfield Cheshire SK10 4TG (GB); CASSIDY, Andrew, Macclesfield Cheshire SK10 4TG (GB)
(86) International application number: PCT/GB2004/003486
(87) International publication number: WO 2005/019452

(56) References cited:
- WO-A-01/73134
- WO-A-99/25873
- US-A- 5 716 785
- HU LIMEI ET AL: "Obtaining reliable information from minute amounts of RNA using cDNA microarrays." BMC GENOMICS [ELECTRONIC RESOURCE]. 21 JUN 2002, vol. 3, no. 16, 21 June 2002 (2002-06-21), pages 1-8, XP002302820 cited in the application
- GUSTINCICH STEFANO ET AL: "Gene discovery in genetically labeled single dopaminergic neurons of the retina." PROCEEDINGS OF THE NATIONAL ACADEMY OF SCIENCES OF THE UNITED STATES OF AMERICA. 6 APR 2004, vol. 101, no. 14, 6 April 2004 (2004-04-06), pages 5069-5074, XP002302942 ISSN: 0027-8424
- KLUR SANDRA ET AL: "Evaluation of procedures for amplification of small-size samples for hybridization on microarrays." GENOMICS, vol. 83, no. 3, March 2004 (2004-03), pages 508-517, XP002302821 ISSN: 0888-7543
- ZHU Y Y ET AL: "REVERSE TRANSCRIPTASE TEMPLATE SWITCHING: A SMART APPROACH FOR FULL-LENGTH CDNA LIBRARY CONSTRUCTION" BIOTECHNIQUES, EATON PUBLISHING, NATICK, US, vol. 30, no. 4, April 2001 (2001-04), pages 892-897, XP001121210 ISSN: 0736-6205 cited in the application
- SHI X ET AL: "5' RACE BY TAILING A GENERAL TEMPLATE-SWITCHING OLIGONUCLEOTIDE" BIOTECHNIQUES, EATON PUBLISHING, NATICK, US, vol. 29, no. 6, December 2000 (2000-12), pages 1192,1194-1195, XP001121212 ISSN: 0736-6205
- SCHMIDT W M ET AL: "Capselect:a high sensitive method for 5'cap-dependent enrichment of full-length cDNA in PCR-mediate analysis of mRNAs" NUCLEIC ACIDS RESEARCH, OXFORD UNIVERSITY PRESS, SURREY, GB, vol. 27, no. 12, 1999, page e31 XP002211678 ISSN: 0305-1048

### SEQUENCE LISTING

<110> AstraZeneca AB
<120> Amplification Method
<130> CCH/101162
<140> GB 0319332.3
   <141> 2003-08-16
<150> GB 0319332.3
   <151> 2003-08-16
<160> 9
<170> PatentIn version 3.2
<210> 1
   <211> 94
   <212> DNA
   <213> Artificial
<220>
   <223> PCR PRIMER
<220>
   <221> misc_feature
   <222> (94)..(94)
   <223> n is a, c, g, or t
<400> 1
   aagcagtggt atcaacgcag agtggccagt gaattgtaat acgactcact atagggaggc      60
   ggtttttttt tttttttttt tttttttttt ttvn                                  94
<210> 2
   <211> 30
   <212> DNA
   <213> Artificial
<220>
   <223> PCR PRIMER
<400> 2
   aagcagtggt atcaacgcag agtacgcggg 30
<210> 3
   <211> 23
   <212> DNA
   <213> Artificial
<220>
   <223> PCR PRIMER
<400> 3
   aagcagtggt atcaacgcag agt 23
<210> 4
   <211> 71
   <212> DNA
   <213> Artificial
<220>
   <223> PCR PRIMER
<220>
   <221> misc_feature
   <222> (71)..(71)
   <223> n is a, c, g, or t
<400> 4
   aagcagtggt atcaacgcag agtaatacga ctcactatag ggagattttt tttttttttt 60
   tttttttttv n 71
<210> 5
   <211> 18
   <212> DNA
   <213> Artificial
<220>
   <223> RNA POLYMERASE PROMOTER
<400> 5
   gcattaaccc tcactaac 18
<210> 6
   <211> 17
   <212> DNA
   <213> Artificial
<220>
   <223> RNA POLYMERASE PROMOTER
<400> 6
   taatacgact cactata 17
<210> 7
   <211> 22
   <212> DNA
   <213> Artificial
<220>
   <223> RNA POLYMERASE PROMOTER
<400> 7
   aatacgactc actataggga ga 22
<210> 8
   <211> 39
   <212> DNA
   <213> Artificial
<220>
   <223> RNA POLYMERASE PROMOTER
<400> 8
   ggccagtgaa ttgtaatacg actcactata gggaggcgg 39
<210> 9
   <211> 17
   <212> DNA
   <213> Artificial
<220>
   <223> RNA POLYMERASE PROMOTER
<400> 9
   atttaggtga cactata 17

## Description

### FIELD OF THE INVENTION

The present invention relates generally to the field of recombinant DNA technology.

In particular, the present invention relates to improved methods for producing amplified heterogeneous populations of RNA from limited quantities of starting RNA.

### BACKGROUND TO THE INVENTION

The field of gene expression profiling has exploded in the last few years, and can now be performed on a genome wide scale. Identification of differentially expressed genes is being used for medical, clinical, and biological research to help understand the molecular mechanisms that underlie biological processes including disease - such as tumourigenesis - differentiation and development. Gene expression profiling can be used across a broad range of applications, for example for the identification of novel targets for therapeutic intervention, identification of potential diagnostic and prognostic markers, to help understand clinical response to drugs and outcome, and identify toxicological responses. DNA arrays of immobilised gene-specific sequences (probes) on a variety of platforms - such as macroarrays, microarrays or high-density oligonucleotide arrays - on different solid supports (e.g., nylon membranes, glass slides or silicon/ceramic chips) are widely applicable in different areas of genomics research.

The most commonly used mechanism for RNA amplification is a T7 based linear amplification method first developed by Van Gelder *et al* (1990), Eberwine *et al* (1992) and Philips and Eberwine (1996), and is described in US 6,291,170, US 5,891,636, US 5,716,785, and US 5,545,522. In this procedure, a synthetic poly(dT) primer containing the phage T7 RNA polymerase promoter is used to prime synthesis of first strand cDNA by reverse transcription. Second strand cDNA is synthesised by limited RNase H digestion, followed by second strand synthesis with *E. coli* DNA polymerase I. Amplified antisense RNA (aRNA) is obtained from *in vitro* transcription of the double-stranded cDNA template using T7 RNA polymerase.

This procedure is the basis for Affymetrix GeneChip oligonucleotide arrays where biotinylated ribonucleotides are incorporated into the aRNA, also known as complementary RNA or (see Affymetrix GeneChip ® Expression Analysis Technical Manual, and Mahadevappa and Warrington (1999)), and is also the basis for glass microarrays (Pabón *et al*, (2001)).

Although this method involves an amplification step with T7 RNA polymerase, it is typically used with 5-25µg total RNA. However, in order to obtain sufficient amounts of amplified RNA (aRNA) for microarray experiments, investigators have resorted to two or more rounds of cDNA synthesis coupled with T7 amplification to generate a representative mRNA profile and this has allowed them to combine microdissection with array technology (Luo *et al* (1999), Ohyama *et al* (2000), Affymetrix Technical Note GeneChip ® Eukaryotic Small sample Target Labelling Assay Version II, and Luzzi et al (2001)).

WO2001/073134 and Wang *et al* (2000) described a method that uses "template switching" and that decreases the amount of starting RNA required, and increases full length message production during ds cDNA production (see below for further background on SMART). The "SMART TM" system (Switch Mechanism At the 5' end of RNA Templates), offered by CLONTECH Laboratories (Palo Alto, CA), is a commercially available template switching system recommended for use in library construction. The Wang *et al* method also differs from previous methods (see Phillips and Eberwine, 1996; and US 5,891,636), in that it used template switching rather than using the Gubler-Hoffman (Gene 25:263-269, 1983) method which employs RNase H and E. coli DNA polymerase I to synthesize the second strand of cDNA.

Wang *et al* (2000) have described a T7-based amplification protocol modified with a SMART^{™} template-switching primer that is used to theoretically generate a full-length double stranded cDNA. The fidelity of aRNA amplified was shown to be comparable between the expression profiles of 1:10000 to 1:100000 of commonly used input RNA, and those observed with conventional polyA+ RNA or total RNA. Hu and co-workers (2002) compared amplified and unamplified samples to evaluate a similar T7 based protocol with a template switching mechanism adopted from Wang *et al*. Their results showed concordance between amplified and non-amplified samples, and four expressed and two differentially expressed genes were vcrified using Northern and Western blotting and immunohistochemical assay.

Incyte Pharmaceuticals have described a method in WO99/025873 where mRNA is first converted to dscDNA with a primer containing an RNA polymerase promoter and at least one priming site. The resultant dscDNA is then enzymatically asymmetrically amplified, by PCR, using a single captureable primer, for example a biotinylated primer, to produce amplified amounts of anti-sense cDNA. Following capture of the captureable cDNA on a solid support, the resultant captured cDNA is converted to ds cDNA, that may then be used for a variety of purposes.

Modifications of the T7-based amplification technology have also been used in two commercially available amplification kits. The RiboAmp™ RNA Amplification Kits (Arcturus) achieves high yields of amplified RNA with a proprietary linear amplification method. This method utilises one or two rounds of T7 based amplification depending on the amounts of starting material. RealArt™ mRNA amplification kit (Artus GmbH) provides a T7 based amplification technology. mRNA is converted to cDNA with an anchored poly(dT) primer, and then second strand synthesis is performed using a proprietary "Box/randomized primer mix" which generates a random representation of the complete cDNA. The randomly primed cDNA is denatured and primed with a T7 promoter/poly(dT) primer. This leads to double stranded cDNA with a T7 promoter at one end that can be used as a template for in *vitro* transcription.

Polymerase chain reaction (PCR) is an extremely powerful technique for amplifying specific nucleic acid sequences as described in US 4,683,202 and US 4,683,195. PCR typically comprises treating separate complementary strands of a target DNA with two oligonucleotide primers to form complementary primer extension products on both strands that can act as templates for synthesising copies of the desired nucleic acid sequences. The separation and synthesis steps are- repeated in an automated system using thermostable polymerases, essentially exponential duplication of the target sequences can be achieved.

Amplification of cDNA by PCR requires the presence of primer binding sites at both cDNA ends. These primer sites can be attached by a number of methods, including a) the addition of a homopolymer tail on the 3' end of the first-strand cDNA (Akowitz & Manuelidis, (1989), Belyavsky et al, (1989), Domec, et al (1990), and Brady, et al (1990)); b) ligation of single-stranded anchor oligonucleotide to the 3' end of the first-strand cDNA (Apte & Siebert, (1993)); c) ligation of single-stranded anchor oligonucleotide sequence to the 5' end of mRNA (Fromont-Racine *et al* (1993), Kato, *et al* (1994), Maruyama & Sugano, S. (1994)); d) ligation of double-stranded adaptors to the 5' end of the double-stranded cDNA (Frohman & Dush, (1988)); and e) addition of amplifier sequence at the 5' and 3' ends of single-stranded cDNA in the first strand synthesis reaction by a template switching mechanism (SMART^{™}, Switching Mechanism At the 5' end of RNA Transcripts, BD Biosciences Clontech) as described in Chenchik *et al* (1998) and Zhu, *et al* (2001)). Template switching is also described in US 5,962,271 and US 5,962,272.

Amplification techniques based on reverse transcription polymerase chain reaction (RT-PCR) have been described for global amplification of mRNAs from single cells (Rappolee, *et al* (1989), Brady, *et al* (1990), Cheng, *et al* (1996), O'Brien, *et al* (1994)). PCR based approaches such as that described by Brady *et al* (1990) have also been employed to look at gene expression by global mRNA amplification followed by cDNA array analysis (Theilgaard-Monch, et al (2001)). However, this method is designed to limit the size of the first strand cDNA to about 300-700 bases, and so corresponding arrays have to be designed such that the arrayed clones contain the 3' ends of each cDNA.

Recently, a rapid and highly optimized global RT-PCR procedure has been described (Iscove, *et al* (2002)) that is also based on methods described previously by Brady *et al* (1990). The procedure involves reverse transcription of a first cDNA strand primed by poly(dT), addition of an poly(dA) tail with terminal transferase, and subsequent exponential amplification using a single poly(dT)-containing primer. Reverse transcription is limited to only a few hundred bases of extreme 3' sequence by limiting deoxynucleotide concentrations and the time of the reaction. These conditions were intended to provide a more uniform likelihood of sampling individual mRNA transcripts and more uniform amplification efficiency across all cycles. This global RT-PCR approach preserved abundance relationships through amplification and yielded reproducible results from the picogram range of total RNA obtainable from single cells.

A PCR-based amplification method, three prime end amplification PCR (TPEA-PCR), has been developed which results in global amplification of the 3' end of all the mRNAs present in a sample (Dixon, *et al* (1998)). PCR amplification occurs between primers incorporated into the first strand cDNA during reverse transcription, and a second strand primer that has a partially degenerate 3' end and is designed to anneal once every kilobase.

Recently, another PCR-based amplification method "balanced PCR" was described by Makrigiorgos, *et al* (2002). Balanced PCR allows a balanced amplification of fragments from two complex DNAs even after three sequential rounds of PCR. Two distinct genomic DNA or cDNA samples are tagged with oligonucleotides containing both a common and a unique DNA sequence. The samples are pooled and amplified in a single PCR tube using the common DNA tag, and therefore there should be no differences in amplification efficiency. The PCR-amplified pooled samples can be separated using the DNA tag unique to each individual genomic DNA sample. The principle of this method has been validated with synthetic DNA, genomic DNA, and cDNA applied on microarrays. By removing the bias of PCR, this balanced PCR approach should allow genetic analysis in minute laser-microdissected tissues, paraffin-embedded archived material, or single cells. However, routine use of this method for amplification of RNAs from biopsies has not yet been reported.

A PCR amplification method based on reverse transcription, followed by random PCR amplification of the cDNA and *in vitro* transcription of the resulting PCR product with T7 RNA polymerase has been described by Roche Applied Science in their Microarray Target Amplification kit (Cat No 3 310 191). In this procedure, nanogram quantities of total RNA are reverse transcribed into cDNA using a modified poly(dT) primer (TAS-T7 poly(dT)24 ). The unique Target Amplification Sequence (TAS) has no homology to any known human sequence, generates the 3' anchor on the cDNA for subsequent PCR amplification. The T7 promoter sequence is added to allow the generation of labelled cRNA targets by *in vitro* transcription. A TAS-(dN)10 primer is used in order to include a 5'amplifier sequence on the cDNA and is used for the initiation of the second strand cDNA synthesis. PCR is then performed with TAS-primer and the optimal numbers of cycles have to be determined. PCR products are then purified and used for *in vitro* transcription.

SMART^{™} generated cDNA has been used for several applications including cDNA library construction, and as hybridisation probes for cDNA and oligonucleotide-based microarrays. SMART^{™} technology coupled with PCR has allowed the use of lower amounts of starting total RNA, although there is only limited data to support the usefulness of this technology in microarray analyses where it is important to maintain the mRNA representation [Spirin, *et al* (1999), Gonzales *et al* (1999), Vernon, *et al* (2000), Livesey, *et al* (2000), Zhumabayeva, *et al*, (2001) and Fink, *et al* (2002)].

The limitation of these technologies is the requirement for relatively large amounts of intact total RNA. By way of example, typical microarray labelling procedures require 0.5-4 µg poly(A) + RNA or 5-50 µg total RNA per microarray. This amount of poly(A) + RNA or total RNA can be obtained from samples of tissue that weigh greater than 50-100 mg, however many samples are significantly less than this, for example many clinical biopsies. A recent pilot study by Assersohn *et al* (2002) showed that only 15% of fine needle aspirates (FNA) from human breast cancers produced sufficient mRNA for expression array analysis.

A simple and robust method that is not restricted by low efficiency and the use of multiple time consuming steps and allows amplification from small amounts of RNA, whilst maintaining sensitivity, reproducibility, and can effectively identify differentially expressed genes is highly desirable in the art.

### SUMMARY ASPECTS OF THE PRESENT INVENTION

The present invention combines the use of template switching with nucleic acid amplification - such as PCR - to generate amplification products representative of an entire RNA population. An RNA polymerase promoter sequence allows transcription-based amplification to be performed on the derived amplification products such that antisense amplified RNA (aRNA) or complementary RNA (cRNA) is produced for subsequent downstream applications.

Advantageously, the RNA generated in accordance with the present invention is antisense and therefore has utility for use on cDNA arrays and oligonucleotide arrays (spotted oligos or solid phase synthesised oligos such as Affymetrix). Commercial or "home-made" arrays are either cDNA or oligo based (typically sense oligos are arrayed) and therefore production of antisense cRNA as we describe herein has utility in either setting. If sense RNA was generated then its use would be limited to cDNA arrays and not the majority of most current oligonucleotide arrays.

The present invention also overcomes the obstacle of limited tissue samples.

In a first aspect, the present invention relates to a cDNA-RNA hybrid comprising a first strand cDNA synthesis substantially hybridised to RNA wherein the cDNA comprises an amplifier sequence and an RNA annealing region operably linked to an RNA polymerase promoter, and wherein at least one non-templated nucleotide at the 3' end of the first strand cDNA is hybridised to a template switching oligonucleotide.

In a second aspect, the present invention relates to a method for amplifying RNA in a sample comprising the steps of: (a) providing a cDNA synthesis oligonucleotide comprising an amplifier sequence and an RNA annealing region operably linked to an RNA polymerase promoter; (b) annealing the RNA annealing region of the cDNA synthesis oligonucleotide to RNA under suitable conditions to produce a cDNA-RNA complex; (c) incubating said cDNA-RNA complex under conditions which permit template-dependent extension of the cDNA synthesis oligonucleotide to generate a cDNA-RNA hybrid; (d) contacting said cDNA-mRNA hybrid with a template switching oligonucleotide under conditions which permit template dependent extension of said cDNA, such that the 3' end of the cDNA comprises a sequence complementary to said template switching oligonucleotide; (e) providing an amplification primer under conditions to generate double stranded amplification products corresponding substantially to the first strand cDNA synthesis, such that the cDNA amplification products comprise a double stranded RNA polymerase promoter; and (f) incubating said cDNA amplification products comprising said double stranded RNA polymerase promoter under conditions that permit *in vitro* transcription to generate amplified RNA.

In a third aspect, the present invention relates to a method for preparing an expression library of a cell or a cell population comprising the steps of: (a) providing a cDNA synthesis oligonucleotide comprising an amplifier sequence and an RNA annealing region operably linked to an RNA polymerase promoter; (b) contacting said cDNA synthesis oligonucleotide with a population of mRNAs from said cell or cell population under conditions to allow hybridisation of said cDNA synthesis oligonucleotide to mRNA to produce a cDNA-mRNA complex; (c) incubating said cDNA-mRNA complex under conditions which permit template-dependent extension of said cDNA synthesis oligonucleotide to generate a cDNA-mRNA hybrid; (d) contacting said cDNA-mRNA hybrid with a template switching oligonucleotide under conditions which permit template dependent extension of said cDNA, such that the 3' end of the cDNA of the cDNA-mRNA hybrid comprises a sequence complementary to said template switching oligonucleotide; (e) contacting an amplification primer with said cDNA-mRNA hybrid under conditions that generate double stranded amplification products corresponding to the first strand cDNA synthesis, such that the double stranded cDNA amplification products comprise a double stranded RNA polymerase promoter; and (f) incubating said double stranded cDNA amplification products comprising said double stranded RNA polymerase promoter under conditions that permit *in vitro* transcription to generate amplified RNA.

In a fourth aspect, the present invention relates to a method of preparing a cDNA library from a collection of mRNA molecules comprising the steps of: (a) providing a cDNA synthesis oligonucleotide comprising an amplifier sequence and an RNA annealing region operably linked to an RNA polymerase promoter; (b) contacting said cDNA synthesis oligonucleotide with the collection of mRNAs under conditions to allow annealing of said cDNA synthesis oligonucleotide to mRNA produce a cDNA-mRNA complex; (c) incubating said cDNA-mRNA complex under conditions which permit template-dependent extension of said cDNA synthesis oligonucleotide to generate a cDNA-mRNA hybrid; (d) contacting said cDNA-mRNA hybrid with a template switching oligonucleotide under conditions which permit template dependent extension of said cDNA of said hybrid, such that the 3' end of the cDNA of the cDNA-mRNA hybrid comprises a sequence complementary to said template switching oligonucleotide; (e) contacting a PCR primer with said cDNA-mRNA hybrid under conditions that generate double stranded amplification products corresponding to the first strand cDNA synthesis, such that the double stranded cDNA amplification products comprise a double stranded RNA polymerase promoter; (f) incubating said double stranded cDNA amplification products comprising said double stranded RNA polymerase promoter under conditions that permit *in vitro* transcription to generate amplified RNA; and (g) preparing a cDNA library from the amplified RNA.

In a fifth aspect, the present invention relates to a method for performing subtractive hybridisation comprising the steps of: (a) providing a cDNA synthesis oligonucleotide comprising an amplifier sequence and an RNA annealing region operably linked to an RNA polymerase promoter; (b) contacting the cDNA synthesis oligonucleotide with a collection of mRNAs under conditions to allow annealing of said cDNA synthesis oligonucleotide to mRNA in said RNA sample to produce a cDNA-mRNA complex; (c) incubating said cDNA-mRNA hybrid with enzyme, dNTPs and buffer under conditions which permit template-dependent extension of said cDNA synthesis oligonucleotide to generate a cDNA-mRNA hybrid; (d) contacting said cDNA-mRNA hybrid with a template switching oligonucleotide under conditions which permit template dependent extension of said cDNA of said hybrid, such that the 3' end of the cDNA of the cDNA-mRNA hybrid comprises a sequence complementary to said template switching oligonucleotide; (e) contacting an amplification primer with said cDNA-mRNA hybrid under conditions to generate double stranded amplification products corresponding to the first stand cDNA synthesis, such that the double stranded cDNA amplification products comprise a double stranded RNA polymerase promoter; (f) incubating said double stranded cDNA amplification products comprising said double stranded RNA polymerase promoter under conditions that permit *in vitro* transcription to generate amplified RNA; (g) contacting said amplified RNA with a single stranded nucleic acid population in the opposite sense to said amplified RNA; (h) providing for the hybridisation of the sequences present in the amplified RNA and the single stranded nucleic acid population; and (i) isolating the nucleic acid population that remains single stranded.

In a sixth aspect, the present invention relates to a method for detecting the expression of a gene of interest comprising the steps of: (a) providing a cDNA synthesis oligonucleotide comprising an amplifier sequence and an RNA annealing region operably linked to an RNA polymerase promoter, wherein the RNA annealing region comprises a sequence that is substantially homologous to the mRNA expressed by the gene of interest; (b) contacting said cDNA synthesis oligonucleotide with a population of mRNAs in a cell or cell population under conditions to allow annealing of said cDNA synthesis oligonucleotide to mRNA to produce a cDNA-mRNA complex; (c) incubating said cDNA-mRNA hybrid under conditions which permit template-dependent extension of said cDNA synthesis oligonucleotide to generate a cDNA-mRNA hybrid; (d) contacting said cDNA-mRNA hybrid with a template switching oligonucleotide under conditions which permit template dependent extension of said cDNA of said hybrid, such that the 3' end of the cDNA of the cDNA-mRNA hybrid comprises a sequence complementary to said template switching oligonucleotide; (e) contacting an amplification primer with said cDNA-mRNA hybrid under conditions to generate double stranded amplification products corresponding to the first stand cDNA synthesis, such that the double stranded cDNA amplification products comprise a double stranded RNA polymerase promoter; (f) incubating said double stranded cDNA amplification products comprising said double stranded RNA polymerase promoter under conditions that permit *in vitro* transcription to generate amplified RNA; and (g) determining the presence or absence of amplified RNA, which amplified RNA is complementary to mRNA corresponding to the gene of interest.

In a seventh aspect, the present invention relates to amplified RNA obtainable by the method according to the second aspect of the present invention.

Advantageously, the amplified RNA could be used in a diagnostic, prognostic or predictive test starting with small limiting amounts of biological or clinical samples, for example but not limited to, biopsies, fine needle aspirates, tissue sections, bronchioalveolar lavage, macrodissected or microdissected tissues.

In an eighth aspect, the present invention relates to an expression library obtainable by the method according to the third aspect of the present invention.

In an ninth aspect, the present invention relates to a cDNA library obtainable by the method according to the fourth aspect of the present invention.

In a tenth aspect, the present invention relates to the use of a cDNA-RNA hybrid according to the first aspect of the present invention in the amplification of RNA.

In an eleventh aspect, the present invention relates to the use of a cDNA-RNA hybrid according to the first aspect of the present invention in the preparation of a cDNA library.

In an twelfth aspect, the present invention relates to the use of a cDNA-mRNA hybrid according to the first aspect of the present invention in subtractive hybridisation.

In an thirteenth aspect, the present invention relates to the use of a cDNA-mRNA hybrid according to the first aspect of the present invention for measuring gene expression.

In an fourteenth aspect, the present invention relates to a kit for the amplification of RNA in a sample comprising: (a) a cDNA synthesis oligonucleotide comprising an amplifier sequence and an RNA annealing region operably linked to an RNA polymerase promoter; (b) a template switching oligonucleotide that has substantially the same sequence as the amplifier sequence; and (c) an amplification primer that has substantially the same sequence as the template switching oligonucleotide.

Other aspects of the present invention are presented in the accompanying claims and in the following description and discussion. These aspects are presented under separate section headings.

### PREFERRED EMBODIMENTS

Preferably, the RNA of the cDNA-RNA hybrid is mRNA.

Preferably, the RNA polymerase promoter is a bacteriophage promoter. More preferably, the bacteriophage promoter is selected from the group consisting of T7, T3 and SP6.

Preferably, the RNA annealing region comprises poly (dT). More preferably, the oligo(T) region is from about 10 to about 30 T residues in length.

Preferably, the 3' end of the RNA annealing region comprises a VN clamp (VN-3'), wherein V is A, G or C and N is A, G, C or T.

Preferably, at least one non-templated nucleotide at the 3' end of the first strand cDNA synthesis is deoxycytidine.

Preferably, at least three non-templated nucleotide at the 3' end of the first strand cDNA synthesis are hybridised to a template switching oligonucleotide.

Preferably, at least three of the non-templated nucleotides at the 3' end of the first strand cDNA synthesis are deoxycytidine nucleotides.

Preferably, the template switching oligonucleotide has at least three guanine residues at its 3' end.

Preferably, the amplifier sequence, amplification primer and the template switching oligonucleotide contain the same sequence.

Preferably, the 3' end of the first strand cDNA synthesis is extended such that it is substantially complementary to the template switching oligonucleotide.

Preferably, the first strand cDNA synthesis is synthesised by a reverse transcriptase.

Preferably, the reverse transcriptase lacks RNaseH activity but retains wild-type polymerase activity. More preferably, the reverse transcriptase is a Moloney Murine Leukemia virus (MMLV) reverse transcriptase or a mutant thereof. Most preferably, the reverse transcriptase is PowerScript™ Reverse Transcriptase (BD Biosciences Clontech).

Preferably, the cDNA-RNA hybrid is incubated with a reverse transcriptase that adds at least one deoxycytidine residue to the 3' end of the first strand cDNA synthesis.

Preferably, the reaction comprises 1 mM dNTPs.

Preferably, the double stranded amplification products are obtained by PCR.

Preferably, the cDNA synthesis oligonucleotide and the PCR primer have the same concentration.

Preferably, the cDNA synthesis oligonucleotide and the PCR primer have a concentration of about 0.5 µM.

Preferably, PCR amplification is performed using the Advantage@ 2 Polymerase mix (BD Biosciences Clontech).

Preferably, the optimum number of cycles to generate the double stranded amplification products is determined by a method comprising the steps of: (a) providing a plurality of samples with a known amount of RNA; (b) performing amplification for a defined number of cycles on the plurality of samples; (c) purifying the double stranded amplification products; (d) providing for the *in vitro* transcription of the purified amplification products; and (e) determining the number of amplification cycles that results in the minimum amount of amplified RNA that is required.

Preferably, the RNA sample is a clinical sample selected from the group consisting of a biopsy, a microdissected tissue, a fine needle aspirate, a flow-sorted cell, a laser captured microdissected cell or a single cell.

Preferably, gene expression is measured using a microarray.

Preferably, the kit according to the fourteenth aspect of the present invention further comprises in a separate container a reverse transcriptase.

Preferably, the kit further comprises in a separate container an RNA polymerase specific to the RNA polymerase promoter of the cDNA synthesis oligonucleotide.

Preferably, the kit further comprises an amplification buffer and one or more amplification enzymes. More preferably, the amplification buffer and the amplification enzyme(s) are PCR amplification buffer and PCR amplification enzyme(s).

Preferably, the kit further comprises a control nucleic acid.

### ADVANTAGES

The present invention has a number of advantages. These advantages will be apparent in the following description. , .

By way of example, the present invention is advantageous since it provides a commercially useful method.

By way of further example, the present invention is advantageous since the method of the present invention is technically simpler and faster than alternative amplification methods, but with equivalent or improved performance over such methods.

By way of further example, the present invention is advantageous since the present invention provides a method for the reproducible and robust amplification of small amounts of total RNA (typically 5ng-50ng or less), or the approximate equivalent of 500-5000 cells, with the possibility of further scope to use even lower amounts.

By way of further example, the present invention is advantageous since it provides a novel method for the amplification of limited amounts of RNA while maintaining the relative representation of mRNAs.

### BRIEF DESCRIPTION OF THE FIGURES

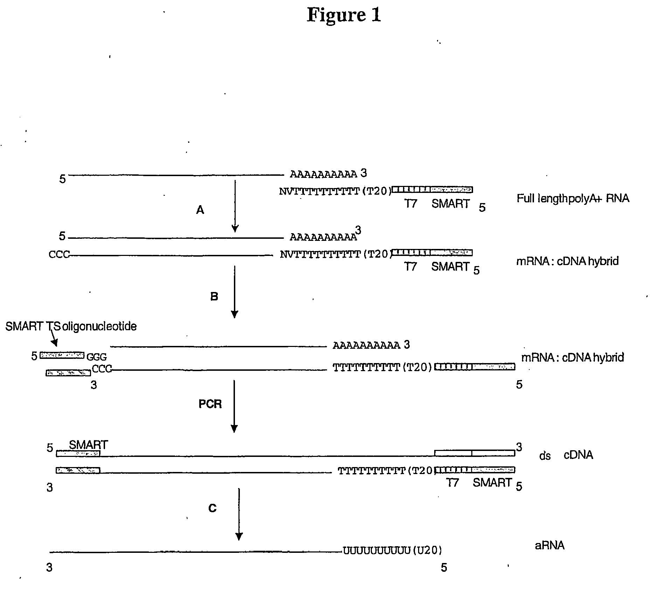

Figure 1 shows a representation of the amplification method according to the present invention (A = First strand synthesis and dC addition by RTase, B = Template switching and extension by RTase, C = In vitro transcription of PCR product, ds = double stranded, aRNA = amplified antisense RNA).

### Step 1

Incubating a sample of poly(A)+RNA or total RNA in the presence of a cDNA synthesis oligonucleotide which can anneal to RNA.

This oligonucleotide has at the 5' end an amplification primer - such as a PCR primer - followed by a T7 promoter sequence and an RNA annealing region. The amplification primer generates the 3' anchor on the cDNA for subsequent amplification and the T7 promoter sequence allows the generation of amplified RNA targets by *in vitro* transcription. An enzyme possessing reverse transcriptase activity is included under suitable conditions to generate an RNA-cDNA hybrid and a template switching oligonucleotide is also included which can provide CAP-dependent extension of full-length cDNA by reverse transcriptase using the template switching oligonucleotide as a template, and thereby adding sequence complementary to the template switching oligonucleotide to the 3'-end of full-length cDNA.

### Step 2

Incubating anchored cDNA:RNA hybrid (ie the population of full-length cDNAs) generated at step 1, with a single amplification primer corresponding substantially or completely to the sequence of the template switching oligonucleotide.

Conditions suitable to generate amplification products are used, the amplification products corresponding to the population of cDNAs present. Advantageously, the amplification conditions are pre-determined to result in sufficient amounts of amplified product for downstream applications but with the minimal number of amplification cycles to minimise any amplification bias. Amplification products are then purified by standard procedures.

### Step 3

The purified amplification products obtained undergo transcription-based amplification using T7 polymerase to generate amplified RNA (aRNA), also known as complementary RNA (cRNA), for downstream applications. The T7 promoter region that is incorporated into the cDNA synthesis oligonucleotide is capable of inducing transcription by T7 polymerase.
Figure 2 shows a comparison of probe synthesis cRNA yields in µg. Bars 17-20 are those prepared according to the standard protocol.
Figure 3 illustrates quality control metrics across arrays. RawQ, BG log and Scaling factor values are shown for each GeneChip hybridisation (A = Raw Q, B = BG log, C = Scaling Factor).
Figure 4. Top: 3' to 5' ratios for the beta-actin (x00351) probe sets. Middle: 3' to 5' ratios for the GAPDH (m33197) probe sets. Bottom: The percentage of probe sets called "Present" as determined by the Affymetrix MAS 5.0 algorithm.
Figure 5 shows the detection of 5', middle, and 3' ends of polyA+ spikes. Top: Lys transcript spiked in at 1pM. Middle: Phe transcript spiked in at 5pM. Bottom: Thr transcript spiked in at 20pM. Each graph shows the signals for the 5'end , middle, and 3' end probe sets for the polyA+ spikes.
Figure 6 represents a clustering dendrogram using Ward's method. The clustered variables are shown along the y axis and the linkage distance along the x axis.
Figure 7 shows a scatter plot (log scale) for ∼22,000 genes. The plot is representative of those obtained for all comparisons.
Figure 8 shows a Spotfire scatter plot showing genes changing more than 2 fold (> | log₁₀ 0.3 |) between samples. The plot is representative of those obtained for all comparisons.

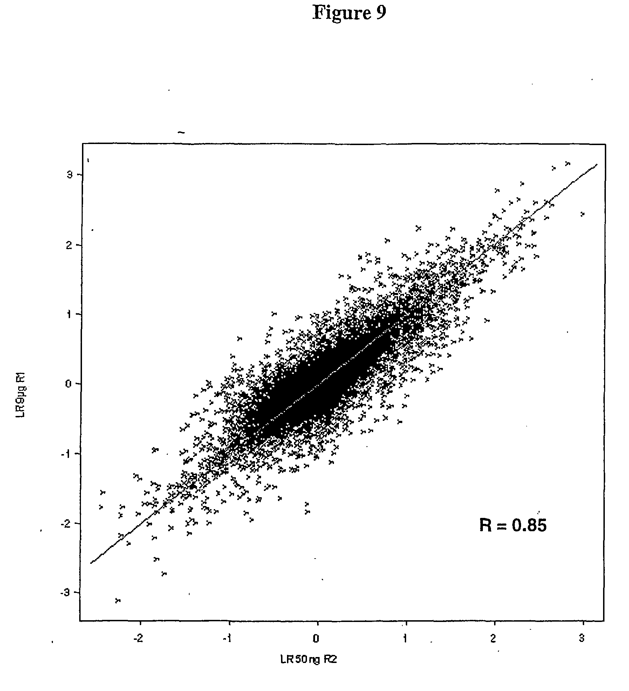

Figure 9 represents a scatter plot comparing log ratios of Set A ("9µg 384 non amp rep1 versus 9µg 842 non amp rep1") versus Set D. Background noise has been removed from the plot i.e. where a signal of <100 was reported for a particular gene across all chip hybridisations. The Pearson correlation value (R) is given for the comparison. Scatter plots comparing Set A versus Set B (R=0.87), Set A versus Set C (R=0.83), Set A versus Set E (R=0.82) and Set A versus Set F (R=0.81) were also prepared, but are not shown. The distribution for Set A versus Set D is representative of those for the other comparisons.
Figure 10 shows Cluster histograms, x axis is cluster and y axis is number of genes. Upper histogram shows all genes in all clusters [Black = genes changing <2 fold, Outline (white) = genes changing >2 fold]. Lower histogram shows only the genes changing >2 fold (y axis enlarged).
Figure 11 shows an example cluster of 53 genes indicating no differential gene expression between samples. Note that amplified and non-amplified probes behave similarly.
Figure 12 shows an example cluster of 21 genes indicating down regulation of gene expression in sample 384. Note that amplified and non-amplified probes behave similarly.
Figure 13 shows an example cluster of 9 genes indicating up regulation of gene expression in sample 842. Note that amplified and non-amplified probes behave similarly.

### DETAILED DESCRIPTION OF THE INVENTION

### OLIGONUCLEOTIDE

The oligonucleotides according to the present invention may be DNA, RNA, chimeric mixtures or derivatives or modified versions thereof that are modified at the base moiety, sugar moiety or backbone and may include other appending groups or labels, so long as they are still capable of functioning in the desired reaction.

By way of example, the oligonucleotide may be a cDNA synthesis oligonucleotide comprising DNA.

By way of further example, the oligonucleotide may be a template switching oligonucleotide comprising DNA.

In a highly preferred embodiment, the template switching oligonucleotide is the template switching oligonucleotide described in US 5,962,271 and 5,962,272.

By way of further example, the oligonucleotide may be an amplification primer - such as a PCR primer - comprising DNA.

The oligonucleotides according to the present invention may be modified so long as they are still capable of functioning in the desired reaction.

The oligonucleotides may be modified at the base moiety, sugar moiety, or phosphate backbone, and may include other appending groups or labels.

The oligonucleotides may comprise at least one modified phosphate backbone - such as phosphorothioate, a phosphorodithioate, a phosphoramidothioate, a phosphoramidate, a phosphordiamidate, a methylphosphonate, an alkyl phosphotriester, and a formacetal or an analogue thereof.

The oligonucleotides may be derived by cleavage of a larger nucleic acid fragment using non-specific nucleic acid cleaving chemicals or enzymes or site-specific restriction endonucleases; or by synthesis by standard methods known in the art, e.g. by use of a commercially available automated DNA synthesiser and standard phosphoramidite chemistry.

Once the desired oligonucleotide is synthesised, it is cleaved from a solid support on which it was synthesised and treated, by methods known in the art, to remove any protecting groups present. The oligonucleotide may then be purified by any method known in the art, including extraction and gel purification. The concentration and purity of the oligonucleotide may be determined by, for example, examining the oligonucleotide on an acrylamide gel, by HPLC, or by measuring the optical density at 260 nm in a spectrophotometer.

In a preferred embodiment of the present invention, the oligonucleotide is a cDNA synthesis oligonucleotide comprising the sequence set forth in SEQ ID No 1:
5'AAGCAGTGGTATCAACGCAGAGTGGCCAGTGAATTGTAATACGACTCACTATA GGGAGGCGG(T)₃₀VN-3'
wherein V is A, G , or C and N is any base.

In another preferred embodiment, the oligonucleotide is a cDNA synthesis oligonucleotide comprising the sequence set forth in SEQ ID No 4:
5'AAGCAGTGGTATCAACGCAGAGTAATACGACTCACTATAGGGAGA(T)₂₄VN-3'
wherein V is A, G , or C and N is any base.

In yet another preferred embodiment of the present invention, the oligonucleotide is a template switching oligonucleotide comprising the sequence set forth in SEQ ID No. 2:

### 5'-AAGCAGTGGTATCAACGCAGAGTACGCGGG-3'

In still another preferred embodiment, the oligonucleotide is an amplification primer comprising the sequence set forth in SEQ ID No. 3:
5'-AAGCAGTGGTATCAACGCAGAGT-3'

The oligonucleotides corresponding to SEQ ID Nos 2 and 3 have been described in US 5,962,271 and 5,962,272.

In the context of the present invention, the cDNA synthesis oligonucleotide comprises an amplifier sequence and an RNA annealing region operably linked to an RNA polymerase promoter.

### AMPLIFIER SEQUENCE

The amplifier sequence in the context of the present invention relates to a portion of the cDNA synthesis oligonucleotide that contains the same or substantially the same sequence as the template switching oligonucleotide and the amplification primer, as described herein.

The amplification primer is able to hybridise to the complementary sequence of the amplifier sequence, such that the second strand cDNA synthesis may be amplified by, for example, PCR.

Preferably, the amplifier sequence is located at the 5' end of the cDNA synthesis oligonucleotide and generally, will not be translated into RNA.

A person skilled in the art will appreciate that any sequence may be used for the amplifier sequence as long as the amplification primer is able to hybridise or substantially hybridise to the complementary sequence of the amplifier sequence.

The substantially identical template switching oligonucleotide and the amplifier sequence at both ends of the cDNA serve as universal priming sites for end-to-end amplification of the cDNA population.

In a preferred embodiment, the amplifier sequence, the amplification primer and the template switching oligonucleotide contain the substantially the same sequence - such as regions with the same sequence.

In another preferred embodiment, the amplifier sequence comprises the sequence set forth in SEQ ID No. 3.

### RNA ANNEALING REGION

As used herein, the term "RNA annealing region" refers to a portion of the cDNA synthesis oligonucleotide that is able to anneal to RNA.

For many applications, it is desirable to preferentially enrich for one type of RNA with respect to other cellular RNAs, such as messenger RNA (mRNA), transfer RNA (tRNA) and ribosomal RNA (rRNA). Advantageously, most mRNAs contain a poly(A) tail at their 3' end which allows them to be enriched by affinity chromatography, for example, using oligo(dT) or poly(U) coupled to a solid support - such as cellulose or Sephadex (Ausubel et al., eds., 1994, Current Protocols in Molecular Biology, vol. 2, Current Protocols Publishing, New York).

Accordingly, in a preferred embodiment of the present invention, the RNA annealing regions comprises poly(dT). Preferably, the poly(dT) is a polythymidylate region comprising about 10 to 30, preferably about 15 to 25, most preferably about 20 T residues, which bind with the poly(A) tail present on the 3' terminus of each mRNA.

If more sequence information is available for a given RNA, then the RNA annealing region may be designed more specifically to hybridise with a more specific population of RNA. Moreover, the RNA annealing region may comprise a collection of RNA annealing regions.

Also, if there is ambiguity in the sequence information, a number of RNA annealing regions may be present. Thus, by way of example, when several possible nucleic acid sequences that encode a protein could be correct based on the protein sequence, a collection of RNA annealing regions containing sequences representing most or all of the possible codon variations may be prepared.

If the sequence information for the desired RNA is known, the RNA annealing region need not reflect the exact sequence of the RNA, and can be "degenerate". Non-complementary bases or longer sequences can be interspersed into the RNA annealing region, provided that the RNA annealing region has sufficient complementarity with the sequence of the strand to be amplified to permit hybridisation.

Typically, the RNA annealing region is located at the 3' end of the cDNA synthesis oligonucleotide and is operably linked to the RNA polymerase promoter.

The term "operably linked" refers to a juxtaposition wherein the RNA annealing region and the RNA polymerase promoter are in a relationship permitting them to function in their intended manner. Thus, in the context of the present invention, the RNA annealing region and the RNA polymerase promoter are in a relationship that permits the RNA annealing region to be expressed from the RNA polymerase promoter.

Advantageously, the 3' end of the RNA annealing region may comprise one or more nucleotides that assist in the priming of mRNA. Preferably, the nucleotides comprise a VN clamp, wherein V is A, G or C and N is A, G, C or T.

### RNA POLYMERASE PROMOTER

Promoter sequences are regions where RNA polymerase binds tightly to DNA and contains the start site and signal for RNA synthesis to begin.

The RNA polymerase promoter will usually comprise between about 15 and 250 nucleotides, preferably between about 15 and 60 nucleotides, most preferably between about 15 and 40 nucleotides, from a naturally occurring RNA polymerase promoter or a consensus promoter region (Alberts et al., in Molecular Biology of the Cell, 2d Ed., Garland, N.Y. (1989)). Native strong promoters typically contain two highly conserved DNA sequences, each about six nucleotides long, which are located upstream from the start site and separated from each other by about 17 nucleotides of unrecognised DNA.

The RNA polymerase that is used for transcription must be capable of binding to the particular RNA polymerase promoter region that is present in the cDNA synthesis oligonucleotide according to the present invention. In practice, any combination of RNA polymerase and RNA polymerase promoter may be used as long as the polymerase has sufficient specificity for that promoter to initiate *in vitro* transcription.

The promoter may be a prokaryotic or a eukaryotic promoter. Preferably, the promoter is a prokaryotic promoter. More preferably, the prokaryotic promoter is a phage or virus promoter. Most preferably, the RNA polymerase promoter is a promoter derived from a bacteriophage, for example, T3, T7 or SP6 polymerase (Chamberlin and Ryan, in The Enzymes, ed. P. Boyer (Academic Press, New York) pp. 87-108 (1982)).

A typical sequence of the T3 RNA polymerase promoter is:
5' GCATTAACCCTCACTAAC 3' (SEQ ID No. 5)

A number of variant T3 promoter sequences are also known, especially those in which the first three bases of the non-template strand (shown above) are 5' TTA 3', rather than AAA. See for example, US 5,037,745.

A typical sequence of the T7 RNA polymerase promoter is:
5' TAATACGACTCACTATA 3' (SEQ ID No. 6)

A number of variant forms of T7 RNA polymerase are also known in the art. By way of example only, further variants of the T7 RNA polymerase promoter are:
5' AATACGACTCACTATAGGGAGA 3' (SEQ ID No. 7)
   and
5' GGCCAGTGAATTGTAATACGACTCACTATAGGGAGGCGG 3' (SEQ ID No. 8)

A typical sequence of the SP6 RNA polymerase promoter is:
5' ATTTAGGTGACACTATA 3' (SEQ ID No. 9)

The RNA polymerase promoter may be a hybrid promoter.

The promoter may additionally include features to ensure or to increase the level of expression.

The most preferred promoter is a T7 RNA polymerase promoter. The very high degree of specificity shown by T7 RNA polymerase for its promoter site (Chamberlin et al., in The Enzymes, ed. P. Boyer (Academic Press, New York) pp. 87-108 (1982)) has made this enzyme a widely used reagent in a variety of recombinant DNA techniques. The natural T7 promoters share a highly conserved sequence covering about bp -17 to about +6 relative to the start of the RNA chain (Dunn and Studier, J. Mol. Biol. 166: 477-535 (1983) and J. Mol. Biol. 175: 111-112 (1984)). The lack of efficient termination signals for T7 polymerase also enable it to make transcripts from almost any DNA (see, Rosenberg et al., Gene 56: 125-135 (1987)).

RNA polymerases are widely available from a number of commercial sources - such as Promega Corporation, Ambion Inc, Enzo Diagnostics Inc., Epicentre Technologies.

The RNA polymerase promoter may be single stranded or double stranded. Advantageously, following the PCR amplification step of the method described herein, the promoter becomes double stranded.

In a highly preferred embodiment, the orientation of the RNA polymerase promoter is such that antisense aRNA is expressed. Advantageously, antisense aRNA may be used in cDNA arrays and oligonucleotide arrays - such as spotted oligonucleotides or solid phase synthesised oligonucleotides eg. Affymetrix. Commercial or "home-made" arrays are either cDNA or oligonucleotide based (typically sense oligonucleotides are arrayed) and therefore production of antisense cRNA as we describe herein has utility in either setting.

### TEMPLATE SWITCHING

Template switching refers to the process of template-dependent synthesis of the complementary strand by an enzyme - such as reverse transcriptase - using two templates in consecutive order and which are not covalently linked to each other by phosphodiester bonds.

The process of template switching is described in Chenchik, et al (1998), Clark, (1988) and Hu & Temin, (1990), US 5,962,271 and US 5,962,272.

Template switching in the context of the present invention is achieved by utilising two of the intrinsic properties of reverse transcriptase namely the ability to add non-templated nucleotides to the 3' end of the first-strand cDNA, and the ability to switch templates.

Preferably, the template switching oligonucleotide comprises more than one residue - at its 3' end that base pairs with the complementary residues that are added at the 3' end of the cDNA. More preferably, the template switching oligonucleotide comprises at least two residues at its 3' end that base pairs with the complementary residues that are added at the 3' end of the cDNA. Most preferably, the template switching oligonucleotide comprises at least three residues at its 3' end that base pairs with the complementary residues that are added at the 3' end of the cDNA.

Preferably, at least one of the residues at the 3' end of the template switching oligonucleotide comprise deoxyguanindine nucleotides. More preferably, at least two of the residues at the 3' end of the template switching oligonucleotide comprise deoxyguanindine nucleotides. More preferably, at least three of the residues at the 3' end of the template switching oligonucleotide comprise deoxyguanindine nucleotides. Most preferably, three of the residues at the 3' end of the template switching oligonucleotide comprise deoxyguanindine nucleotides.

Reverse transcriptase replicates to the 5' end of the mRNA then switches templates and continues to replicate to the end of the template switching oligonucleotide. The resulting first-strand cDNA synthesis contains the complete 5' ends of the mRNA's as well as sequences complementary to the template switching oligonucleotide.

The substantially identical template switching oligonucleotide and the amplifier sequence at both ends of the cDNA serve as universal priming sites for end-to-end cDNA amplification of the cDNA population.

A person skilled in the art will appreciate that any sequence may be used for the template switching oligonucleotide as long as it is identical or substantially identical to the amplification primer. Accordingly, in a preferred embodiment of the present invention the template switching oligonucleotide has substantially the same sequence as the amplifier sequence.

The 3' end of the first strand cDNA synthesis is extended such that it is complementary or substantially complementary to the template switching oligonucleotide. Advantageously, an amplification primer is used that is able to hybridise to the sequence that is complementary to the template switching oligonucleotide. A DNA polymerase is then able to extend from the 3' end of the hybridised or substantially hybridised amplification primer, thereby resulting in a second strand cDNA synthesis.

### cDNA-RNA HYBRID

In the context of the present invention, a cDNA-RNA hybrid refers to a hybrid that is formed between RNA and a first-strand cDNA synthesis in which the cDNA is extended such that the first-strand cDNA synthesis is complementary or substantially complementary to the RNA.

Typically, at least one non-templated nucleotide at the 3' end of the first strand cDNA is hybridised to a template switching oligonucleotide in the cDNA-RNA hybrid.

Preferably, at least one non-templated nucleotide at the 3' end of the first strand cDNA synthesis is deoxycytidine. More preferably, at least two non-templated nucleotides at the 3' end of the first strand cDNA synthesis are hybridised to a template switching oligonucleotide. More preferably, at least three non-templated nucleotides at the 3' end of the first strand cDNA synthesis are hybridised to a template switching oligonucleotide.

Preferably, at least one of the non-templated nucleotides at the 3' end of the first strand cDNA synthesis are deoxycytidine nucleotides. More preferably, at least two of the non-templated nucleotides at the 3' end of the first strand cDNA synthesis are deoxycytidine nucleotides. Most preferably, at least three of the non-templated nucleotides at the 3' end of the first strand cDNA synthesis are deoxycytidine nucleotides.

Preferably, the hybrid is a cDNA-mRNA hybrid.

Typically, the first strand cDNA synthesis is catalysed by a reverse transcriptase using the RNA of the cDNA-mRNA as a template.

### cDNA-RNA COMPLEX

A cDNA-RNA complex refers to a complex that is formed between RNA and a cDNA synthesis oligonucleotide in which the RNA annealing region of the cDNA synthesis oligonucleotide is hybridised or substantially hybridised to a complementary or substantially complementary RNA.

The RNA template-dependent extension of the cDNA synthesis oligonucleotide results in extension of the cDNA synthesis oligonucleotide such that the first-strand cDNA synthesis is complementary or substantially complementary to RNA, thus forming a cDNA-mRNA hybrid.

Preferably, the complex is a cDNA-mRNA complex.

### SAMPLE

A sample in the context of the present invention may be any entity that comprises RNA.

Any RNA, in purified or non-purified form, may be utilised in the method of the present invention, provided that it contains or is suspected to contain the RNA that is of interest. The desired RNA may be a minor or a major fraction of a complex mixture. Accordingly, the present invention is useful not only for producing large amounts of one specific nucleic acid sequence, but also for amplifying simultaneously one or more different specific nucleic acid sequences.

The RNA - such as cloned RNA or total RNA - may be obtained from any prokaryotic or eukaryotic source, for example, bacteria, yeast, viruses, organelles, and higher organisms such as plants or animals. RNA may be extracted from blood, tissue material or cells by a variety of techniques such as those described in Maniatis *et al., supra*.

Accordingly, the sample may be or may be derived from biological material.

The sample may be a clinical sample - such as a biopsy, microdissected tissue or laser-captured cells. Preferably, the sample is a small sample, for example, a small biopsy, a fine needle aspirate, a macrodissected tissue, a flow-sorted cell, a laser captured microdissected cell or a small number of cells - such as a single cell.

Advantageously, the present invention provides a method for the reproducible and robust amplification of small amounts of total RNA. Preferably, the present invention is able to amplify 5-50 ng of total RNA, more preferably, the present invention is able to amplify 5-25ng total RNA, most preferably, the present invention is able to amplify 5 ng or less total RNA with the possibility of further scope to use even lower amounts.

5-50ng total RNA equates to the approximate equivalent of 500-5000 cells.

Total cellular RNA, cytoplasmic RNA, or poly(A)+ RNA may be used. Methods for preparing total and poly(A)+ RNA are well known and are described generally in Sambrook et al. (1989, Molecular Cloning--A Laboratory Manual (2nd Ed.), Vols. 1-3, Cold Spring Harbor Laboratory, Cold Spring Harbor, N.Y.) and Ausubel et al., eds. (1994, Current Protocols in Molecular Biology, vol. 2, Current Protocols Publishing, New York).

Preferably, total RNA is prepared by the techniques described in Chirgwin et al, (1987), Chomczynski & Sacchi (1987), Sambrook et al, (1989), or Farrell Jr. (1993), and a number of high quality commercial kits are also available. More preferably, total RNA used is prepared using the guanidinium thiocyanate method of Chirgwin et al, (1987).

The integrity of total RNA may be checked using various methods that are known in the art. By way of example, the RNA may be analysed using RNA gel electrophoresis (eg formaldehyde/ agarose gel), or Agilent LabChip. For mammalian total RNA, two bands at approximately 4.5 and 1.9 kb should be visible; these bands represent 28S and 18S ribosomal RNA respectively, and the ratio of intensities of these bands should typically be 1.5-2.5:1.

RNA purification kits for microscale RNA preparation are available from a number of commercial suppliers (for example Absolutely RNA™ Nanoprep, Stratagene; PicoPure™, Arcturus; RNeasy®, Qiagen; RNAqueous™ Microkit, Ambion).

Generally, the RNA samples are immediately snap-frozen in liquid nitrogen and then stored at -80°C until RNA extraction.

Typically, the total RNAs are treated with RNase-free DNaseI, which may be obtained from various manufacturers - such as Ambion's DNA-free™ kit.

### cDNA SYNTHESIS

In accordance with the present invention, the first and second strand cDNA synthesis may be performed in separate tubes. Advantageously, the first and the second strand synthesis are performed in the same tube which enhances the synthesis procedure, maximises recovery of cDNA and makes the method simpler and quicker to perform.

The cDNA synthesis oligonucleotide for first strand cDNA synthesis may be hybridised to RNA in a suitable buffer at a temperature between about 60°C and 90°C, preferably about 70°C for about 5 minutes, followed by cooling to about 4 °C, before the reverse transcriptase is added.

Following the hybridisation of the cDNA synthesis oligonucleotide to RNA, a first cDNA strand is synthesised. This first strand of cDNA is preferably produced through the process of reverse transcription, wherein DNA is made from RNA, utilising reverse transcriptase following methods that are familiar to a person skilled in the art.

Advantageously, any reverse transcriptase may be used in the present invention as long as the enzyme adds deoxyribonucleotides to the 3' terminus following extension (Varmus, Science 240: 1427-1435 (1988)) and the enzyme lacks RNase H activity.

Preferably, the reverse transcriptase lacks RNaseH activity but retains wild-type polymerase activity such that longer cDNA's can be synthesised. More preferably, the reverse transcriptase is Moloney Murine Leukemia virus (MMLV) reverse transcriptase or a mutant thereof. Most preferably, the reverse transcriptase is PowerScript™ Reverse Transcriptase (BD Biosciences Clontech).

The amount of reverse transcriptase employed may vary as will be appreciated by a person skilled in the art. The reverse transcription is performed by incubation for, for example, approximately 1 hour with reverse transcriptase at an appropriate temperature, which must be in a temperature range in which the reverse transcriptase retains enzyme activity.

The reaction may be performed between 37°C and 55°C, preferably between 37°C and 42°C.

Most preferably, the reaction is performed at optimal enzyme activity - such as at about 42 °C.

The reverse transcription reaction may be terminated by heating the reaction mixture to 95 °C for about 5 minutes to inactivate the enzyme, optionally, followed by chilling on ice.

The first strand cDNA synthesis step may be modified by including dNTP's - such as 1 mM dNTP's - in the initial step in the procedure where the RNA is annealed to the cDNA synthesis oligonucleotide, by, for example heating to 70°C for 2 min. This inclusion of dNTPs has previously been shown to increase the efficiency in another application, namely RT-PCR reactions (Huang, *et al*, (2000)). Advantageously, this modification may increase the yield of cRNA, from defined small amounts of starting total RNA. Although the mechanism for this observation is not known, without wishing to be bound by any particular theory, it may be due to stabilisation of RNA-primer hybridisation, and/or may help stabilisation of the template switching mechanism.

Advantageously, this modification allows a reduced number of amplification (eg. PCR) cycles with limited starting material, and may therefore help minimise any distortions to the mRNA distribution.

Typically, a single cycle of reverse transcription is carried out. More than one cycle of reverse transcription may be performed (with denaturation in between cycles).

### AMPLIFICATION

"Amplification" refers to a process for multiplying nucleic acid strands *in vitro.*

In a highly preferred embodiment, the amplification method of the present invention is used for multiplying DNA strands - such as cDNA - *in vitro.*

An exemplary technique is PCR, which exponentially amplifies nucleic acid molecules.

PCR is described in US 4,683,195 and US 4,683,202. PCR consists of repeated cycles of DNA polymerase generated primer extension reactions. The target DNA is heat denatured and two oligonucleotides, which bracket the target sequence on opposite strands of the DNA to be amplified, are hybridised. These oligonucleotides become primers for use with DNA polymerase. The DNA is copied by primer extension to make a second copy of both strands. By repeating the cycle of heat denaturation, primer hybridisation and extension, the target DNA can be amplified a million fold or more in about two to four hours. PCR is a molecular biology tool which must be used in conjunction with a detection technique to determine the results of amplification. An advantage of PCR is that it increases sensitivity by amplifying the amount of target DNA by 1 million to 1 billion fold in approximately 4 hours.

PCR may be used in the methods of the present invention as follows. A DNA polymerase - such as *Taq* DNA polymerase - is added to the reaction in addition to a single PCR primer that comprises substantially the same sequence as the amplifier sequence of the cDNA synthesis oligonucleotide.

In a preferred embodiment, the polymerase that is used is Advantage® 2 Polymerase Mix (BD Biosciences Clontech), which allows efficient and accurate amplification of cDNA templates by long-distance PCR (Barnes, 1994). The Advantage® 2 Polymerase Mix contains TITANIUM^{™} Taq DNA Polymerase, a nuclease-deficient N-terminal deletion of Taq DNA Polymerase, and a minor amount of a proofreading polymerase. Advantage® 2 Polymerase Mix also contains TaqStart™ Antibody (BD Biosciences Clontech) to provide automatic hot-start PCR (Kellogg et al., 1994) and reduce non-specific priming of template. This combination allows efficient amplification of full-length cDNAs with a significantly lower error rate than that of conventional PCR (Barnes, 1994).

The single PCR primer hybridises substantially to the 3' end of the first strand cDNA synthesis (after denaturation) which corresponds to the complementary sequence of the template switching oligonucleotide. The DNA polymerase extends from the 3' end of the PCR primer resulting in a complementary cDNA second strand synthesis. In subsequent rounds of PCR, the single PCR primer is able to hybridise to the 3' end of the first strand of the cDNA molecule (and all the amplified copies of the first strand) and to the 3' end of the second strand of the cDNA molecule (and all the amplified copies of the second strand), resulting in amplification.

The primer is preferably a single stranded oligodeoxynucleotide. The primer must be sufficiently long to act as a template for the synthesis of extension products in the presence of the replicating enzyme. The exact lengths of the primers and the quantities used will depend on many factors, including temperature, degree of homology and other conditions.

For example, when amplifying a specific sequence, the oligonucleotide primer typically contains between about 10 and 50 nucleotides, preferably 15-25 or more nucleotides, although it may contain fewer nucleotides, depending. For other applications, the oligonucleotide primer is typically, but not necessarily, shorter, e.g., 7-15 nucleotides. Such short primer molecules generally require cooler temperatures to form sufficiently stable hybrid complexes.

In a preferred embodiment of the present invention, the PCR primer and the cDNA synthesis oligonucleotide are provided at the same concentration in the reaction.

In another preferred embodiment, the cDNA synthesis oligonucleotide and the PCR primer have a concentration of about, for example, 0.5 µM.

The oligonucleotide primers may be prepared using any suitable method, such as, for example, the well known phosphotriester and phosphodiester methods, or automated embodiments thereof. One method for synthesising oligonucleotides on a modified solid support is described in US 4,458,066. It is also possible to use a primer which has been isolated from a biological source (such as a restriction endonuclease digest).

In a preferred embodiment, the PCR primer comprises the sequence set forth in SEQ ID No. 3.

PCR amplification is performed using methods that are well known in the art. By way of example only, the thermal cycling parameters of the PCR reactions may comprise 60 sec at 95°C for hot start, followed by 3 step cycling for a pre-determined numbers of cycles of denaturation for 15 sec at 95°C, annealing for 30 sec at 65°C, and extension for 6 min at 68°C. Reactions may then be held at 4°C in the thermal cycler until purification.

Advantageously, an improved method for determining the optimal number of amplification cycles that are required from a given amount of starting material may be utilised. For any amplification method - such as PCR - one of the key factors is to perform the minimum number of cycles (for a given amount of starting material) that will result in sufficient target for the intended downstream application. If too many cycles are performed, then it is likely that the representation of RNAs - such as mRNAs - could be biased as the amplification reactions are unlikely to be identical for each RNA template in the complex mixture, and the amplification reaction may also reach a plateau. If too few cycles are performed then insufficient amplification product may be obtained for subsequent applications.

In the method described here, for a given amount of starting total RNA (eg 50ng or 5ng), the minimum numbers of amplification cycles that are required are determined empirically such that when the entire amplification products are then used for *in-vitro* transcription (IVT) reactions to generate cRNA, sufficient cRNA for the intended downstream application is obtained. This can be done by starting with a given amount of total RNA (eg 5ng or 50ng) by setting up identical reactions and performing an amplification reaction for defined numbers of cycles (or by removing aliquots from an amplification reaction for analysis and performing additional cycles on the remainder of the amplification reaction). The entire amplification products from each reaction (for example 50ng starting total RNA that has undergone PCR thermal cycling for 9, 10, 11, 12, 13 or 14 cycles) is purified and used for IVT. The number of cycles that gives the minimum amount of cRNA that is sufficient for the downstream application is then determined. This number of cycles can then be used routinely for similar studies with other RNA's at the same starting concentrations.

Advantageously, the amplification step can also be enhanced by addition of dNTP's in the initial step in the procedure where the RNA is heated to 70°C for 2 minutes.

In a preferred embodiment, about 1 mM dNTPs are added to the RNA/primer mix when the samples are being denatured prior to reverse transcription.

This means that less amplification is required with limited amounts of starting material and therefore help minimise any distortions to the mRNA distributions.

The amplification products that are obtained will typically be purified. This may be achieved using various methods that are known in the art. By way of example, PCR products may be purified using QIAGEN Qiaquick columns as per manufacturer's instructions.

### SUBSTANTIALLY

The term "substantially" when used in relation to annealing or hybridisation, means that an oligonucleotide - such as a primer - should be sufficiently complementary to hybridise or anneal to its respective nucleic acid.

The oligonucleotide sequence need not reflect the exact sequence of its respective nucleic acid, and can, in fact, be "degenerate". Non-complementary bases or other sequences may be interspersed into the oligonucleotide or the nucleic acid, provided that the oligonucleotide sequence has sufficient complementarity with the sequence to permit hybridisation. Thus, by way of example, the primers used for PCR amplification may be selected to be "substantially" complementary to the specific sequence to be amplified.

### HYBRIDISATION

As used herein, the term "hybridisation" refers to the process by which a strand of nucleic acid joins with a complementary strand through base pairing as well as the process of amplification as carried out in, for example, polymerase chain reaction (PCR) technologies.

The present invention encompasses the use of nucleotide sequences that are capable of hybridising to nucleotide sequences.

### TRANSCRIPTION

The PCR reaction step described above results in a double stranded T7 RNA polymerase promoter that is operably linked to a double stranded DNA sequence.

By utilising a double stranded T7 promoter, the DNA sequence that is operably linked to the promoter may be transcribed into RNA. Various methods for *in vitro* transcription are well known in the art and many commercial kits are readily available.

By way of example only, the ENZO ® BioArray™ HighYield™ RNA Transcript Labelling Kit, Affymetrix (900182) may be used. The necessary reagents for the *in vitro* transcription are combined with the PCR reactions and *in vitro* transcription is performed at an appropriate time and temperature - such as 37°C for 5hrs. The incubation time may be varied depending upon how many transcripts it is desired to generate.

Depending on the ultimate use of the RNA, the necessary ribonucleotide triphosphates will be included in the transcription reaction mixture. One or more of the ribonucleotides may be labelled, with for example, a radioactive label, biotin, or the like. A wide variety of labelling techniques are well known to those skilled in the art and may be used in accordance with standard procedures, as described in US 4,755,619, for example.

Once the RNA transcripts have been obtained, various well known procedures may be employed for their processing. The transcripts may be removed from the reaction mixture and purified using various methods known in the art - such as the RNeasy® Mini columns (QIAGEN) as per manufacturer's instructions. The aRNA may be used as template for cDNA synthesis and subjected to PCR to further expand desired sequences. The aRNA may be used unmodified for further cloning, expression, use as probe or driver nucleic acid in subtractive hybridisation and the like.

### AMPLIFIED RNA

As used herein, the term amplified RNA (aRNA) is used interchangeably with the term complementary RNA (cRNA).

aRNA refers to the amplified antisense RNA that is obtained from *in vitro* transcription of the double-stranded cDNA template using an RNA polymerase.

### LABEL

In accordance with the present invention, amplified RNA may be labelled during *in vitro* transcription to facilitate its detection/use in subsequent steps.

The amplified RNA may be directly labelled with any label that is known in the art, including, but not limited to, radioactive labels, fluorophores, chemiluminescent molecules, or enzymatic markers - such as those that produce a detectable signal when a particular chemical reaction is conducted - and the like.

Alternatively, ribonucleotides may be obtained which are labelled with, for example, biotinylated CTP and UTP, where these ribonucleotides will become incorporated in the amplified RNA. The biotin may then be used for binding to avidin, which is labelled with an appropriate label capable of providing for detection. Other modified ribonucleotides - such as cyanine 3 and cyanine 5 CTP and UTP, or aminoallyl UTP can be readily incorporated into amplified RNA. A wide variety of labelling techniques are well known to those skilled in the art.

Labelling of RNA may be accomplished by including one or more labelled NTPs in the *in vitro* transcription reaction mixture. NTPs may be directly labelled with a radioisotope, such as ³²P, ³⁵S, ³H. NTPs may be directly labelled with a fluorescent label - such as fluorescein isothiocyanate, lissamine, Cy3, Cy5, and rhodamine 110.

RNA may also be indirectly labelled by incorporating a nucleotide linked covalently to a hapten or to a molecule - such as biotin - to which a labelled avidin molecule may be bound, or digoxygenin, to which a labelled anti-digoxygenin antibody may be bound. RNA may be labelled with labelling moieties during chemical synthesis or the label may be attached after synthesis by methods known in the art.

Often it is desired to compare gene expression in two different populations of cells, perhaps derived from different tissues or perhaps exposed to different stimuli. Such comparisons are facilitated by labelling the RNAs from one population with a first fluorophore and the RNAs from the other population, with a second fluorophore, where the two fluorophores have distinct emission spectra. Again, Cy3 and Cy5 are particularly preferred fluorophores for use in comparing gene expression between two different populations of cells.

### NUCLEOTIDE SEQUENCE

As used herein, the term "nucleotide sequence" is synonymous with the term "polynucleotide".

Aspects of the present invention involve the use of nucleotide sequences, which may be available in databases.

The nucleotide sequence may be DNA or RNA of genomic or synthetic or recombinant origin. The nucleotide sequence may be double-stranded or single-stranded whether representing the sense or antisense strand or combinations thereof.

The nucleotide sequence may be prepared by use of recombinant DNA techniques (e.g. recombinant DNA).

The nucleotide sequence may be the same as the naturally occurring form, or may be derived therefrom.

### VARIANTS/HOMOLOGUES/DERIVATIVES

In the context of the present invention, reference to nucleic and amino acid sequences includes mutants, variants, homologues, derivatives or fragments thereof. Moreover, reference to a particular polypeptide includes mutants, variants, homologues, derivatives or fragments thereof which have the activity of the naturally occurring polypeptide and includes those polypeptides that differ from naturally occurring forms by having amino acid deletions, substitutions, and additions.

Thus, the present invention encompasses the use of variants, homologues and derivatives of nucleotide and amino acid sequences. Here, the term "homologue" means an entity having a certain homology with amino acid sequences or nucleotide sequences. Here, the term "homology" can be equated with "identity".

In the present context, an homologous sequence is taken to include an amino acid sequence which may be at least 75, 85 or 90% identical, preferably at least 95 or 98% identical to the subject sequence. Although homology can also be considered in terms of similarity (i.e. amino acid residues having similar chemical properties/functions), it is preferred to express homology in terms of sequence identity.

An homologous sequence is taken to include a nucleotide sequence which may be at least 75, 85 or 90% identical, preferably at least 95 or 98% identical to the subject sequence.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Homologous substitution (substitution and replacement are both used herein to mean the interchange of an existing amino acid residue, with an alternative residue) may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid#, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid ^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues.

### USES

It will be appreciated that the amplified RNA produced in accordance with the present invention represents a useful intermediate for a wide variety of downstream applications.

### cDNA libraries

The amplified RNA may facilitate the construction of complex cDNA libraries from extremely limited amounts of tissue.

The amplified RNA can be easily converted into double stranded cDNA using various methods that are well known in the art.

Optionally the double stranded cDNA that is generated may be inserted into a vector. This allows the recombinant DNA molecules comprising the cDNA library to be introduced into host cells - such as eukaryotic and prokaryotic hosts.

### Ribonucleotide probes

The amplified RNA may also be used for the production of specific ribonucleotide probes without prior cDNA cloning into riboprobe vectors.

### Subtractive hybridisation

Furthermore, the amplified RNA provides a source of large amounts of single-stranded, anti-sense material for use as driver in subtractive hybridization. For example, two nucleic acid populations, one sense, and one anti-sense, may be allowed to mix together with one population present in molar excess (driver). Sequences present in both populations will form hybrids, whereas sequences present in only one population remain single-stranded. Thereafter, various well known techniques are used to separate the unhybridised molecules representing differentially expressed sequences. Accordingly, the amplified RNA may also be applied to improve methods of detecting and isolating nucleic acid sequences that vary in abundance among different populations, such as in comparing mRNA expression among different tissues or within the same tissue according to physiologic state. Examples of subtractive hybridisation technologies include Suppression Subtractive Hybridisation technology (US 5,565,340), representation difference analysis (US 5,436,142); and linker capture subtraction (Anal. Biochem. (1996) 237:109-114).

### Anti-sense RNA

Anti-sense RNA has a wide variety of uses in both analytical research and therapeutics. Anti-sense RNA functions in several prokaryotic systems to regulate gene expression. Similarly, anti-sense RNA can regulate the expression of many eukaryotic genes. This permits blocking expression of undesirable genes. Therapeutic use of anti-sense RNA therefore involves in *vitro* synthesis of anti-sense RNA with subsequent introduction into the subject (see, generally, Melton, Antisense RNA and DNA, Cold Spring Harbor (1988)).

### Arrays

The application of array technology is often limited because substantial amounts of RNA are required for target preparation. The present invention is therefore particularly suited to the generation of targets for array - such as microarray analysis, in particular, Affymetrix arrays.

Array technology and the various techniques and applications associated with it are described generally in numerous textbooks and documents. These include Lemieux et al., (1998), Molecular Breeding 4, 277-289, Schena and Davis. Parallel Analysis with Biological Chips. in PCR Methods Manual (eds. M. Innis, D. Gelfand, J. Sninsky), Schena and Davis, (1999), Genes, Genomes and Chips. In DNA Microarrays: A Practical Approach (ed. M. Schena), Oxford University Press, Oxford, UK, 1999), *The Chipping Forecast (*Nature Genetics special issue; January 1999 Supplement), Mark Schena (Ed.), Microarray Biochip Technology, (Eaton Publishing Company), Cortes, 2000, The Scientist 14[17]:25, Gwynne and Page, Microarray analysis: the next revolution in molecular biology, Science, 1999 August 6; and Eakins and Chu, 1999, Trends in Biotechnology, 17, 217-218.

### Detection

In a further aspect, the methods of the present invention may be used to identify one or more sequences in a sample by detecting the amplified sequences in the amplified RNA.

### Differential amplification

In a further aspect, the present invention may be used for the detection of differentially expressed genes. It is therefore useful for determining the relative levels of a given sequence relative to other sequences.

Such methods may be particularly useful in, for example, molecular diagnostics, where diagnosis is not based upon the presence or absence of a sequence, but on the relative levels of a given sequence.

### KITS

The materials for use in the methods of the present invention are ideally suited for preparation of kits.

Such a kit may comprise containers, each with one or more of the various reagents (typically in concentrated form) utilised in the methods, including, for example, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more oligonucleotides of the present invention.

Oligonucleotides in containers can be in any form, e.g., lyophilized, or in solution (e.g., a distilled water or buffered solution), etc. Oligonucleotides ready for use in the same amplification reaction can be combined in a single container or can be in separate containers.

The kit optionally further comprises in a separate container an RNA polymerase specific to the RNA polymerase promoter, and/or a buffer for PCR, and/or a DNA polymerase.

The kit optionally further comprises a control nucleic acid.

A set of instructions will also typically be included.

### GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

The invention will now be further described by way of Examples, which are meant to serve to assist one of ordinary skill in the art in carrying out the invention and are not intended in any way to limit the scope of the invention.

### EXAMPLES

### Example 1

### Materials and Methods

BD Biosciences Clontech's SMART^{™} technology allows PCR amplification of 1st strand DNA by incorporating a priming site at the 5' and 3' ends via the template switching mechanism. The primer (SEQ ID No. 1):
5*'*AAGCAGTGGTATCAACGCAGAGT*ggccagtgaattgtaatacgactcactatagggaggcgg*(T)₃₀VN -3'
a 94-mer, is used to prime cDNA synthesis. The upper case region at the 5' end, is identical to the 5' PCR Primer II A provided in BD Biosciences Clontech's SMART^{™} PCR cDNA Synthesis Kit and this sequence generates the 3' anchor on the cDNA for subsequent PCR amplification.. The lower case region is identical to the T7 promoter sequence currently used in the Affymetrix cDNA synthesis primer. The T7 promoter sequence is added to allow the generation of labelled cRNA targets by *in vitro* transcription. The (T)30 region will bind to poly A tail of messenger RNAs and the 3'-terminal VN clamp (where V is A, G , or C and N is any base) helps ensure priming of mRNA. This oligonucleotide was purified by polyacrylamide gel electrophoresis before use.

"SMART^{™} II A" Oligonucleotide (10 uM, BD Biosciences Clontech) (SEQ ID No. 2):
5'-AAGCAGTGGTATCAACGCAGAGTACGCGGG-3'
and "5' PCR Primer II A" (10 uM, BD Biosciences Clontech) (SEQ ID No. 3):
5'-AAGCAGTGGTATCAACGCAGAGT-3'
are identical sequence to those in BD Biosciences Clontech's SMART^{™} PCR cDNA Synthesis Kit.

All reactions are performed in 0.2ml thin-walled PCR tubes.

### RNA

The RNA used in these studies was prepared using guanidinium thiocyanate method [Chirgwin *et al,* (1987)], with all the usual precautions for handling RNA. Cytoplasmic RNA or PolyA+ RNA could also be used in this technique. Integrity of total RNA should be checked by analysis of a sample on RNA gel electrophoresis (eg formaldehyde/ agarose gel), or Agilent LabChip. For mammalian total RNA, two bands at approximately 4.5 and 1.9 kb should be visible; these bands represent 28S and 18S ribosomal RNA respectively, and the ratio of intensities of these bands should be 1.5-2.5:1. RNA purification kits for microscale RNA preparation are available from a number of commercial suppliers (for example Absolutely RNA™ Nanoprep, Stratagene; PicoPure™, Arcturus; RNeasy®, Qiagen; RNAqueous™ Microkit, Ambion).

Total RNAs for the studies reported here were isolated from two human bladder tumour biopsies that were obtained from an external collaboration between AstraZeneca and Institut Curie, Paris and Henri Mondor Hopital, Creteil, Paris. These bladder transitional cell carcinoma biopsies were from a T3 grade 3 (sample 384) and a superficial Ta grade 1 (sample 842). Bladder biopsies were surgically removed by transurethral resection by Dominique Chopin at Henri Mondor Hopital, Creteil, Paris. Samples were immediately snap-frozen in liquid nitrogen and then stored at -80oC until RNA extraction. Total RNA was purified by guanidinium thiocyanate extraction followed by cesium chloride gradient ultracentrifugation (Chirgwin et al, (1987)) at Institut Curie, Paris, quantitated by A260 measurement and the integrity checked by RNA gel electrophoresis. Total RNAs were treated with RNase-free DNaseI (Ambion, DNA-free™ kit) according the manufacturers instructions such that final concentrations were 1ug/ul. . As a reference, the targets were also prepared following the standard protocol (Affymetrix *GeneChip*® *Expression Analysis Technical Manual*) starting with 9ug of the same total RNAs.

DNase I-treated total RNAs were then diluted with RNase-free water, and 5ng or 50ng of total RNA were then used in each amplification reaction.

4 RNA control sense polyA+ spikes were added to the total RNAs using a (20x) stock consisting of Lys, Phe and Thr from B.subtilis (ATCC) and kanamycin positive control polyA+RNA (Promega Corporation, Cat C1381). Plasmids for Lys, Phe and Thr were obtained from ATCC [pGIBS-Lys, ATCC 87482; pGIBS-Phe, ATCC 87483; and pGIBS-Thr ATCC 87484] and sense RNA transcripts were generated and purified as described in Affymetrix GeneChip® Expression Analysis Technical Manual [http://www.affymetrix.com/].

Lys, Phe, Thr and kanamycin spikes were added at final concentrations of 1, 5, 20 and 1pM respectively to the non-amplified RNA samples.

Table (1) gives a summary of the probes synthesised and their respective cRNA yields. Note that for the conventional Affymetrix standard protocol, RNA / T7(dT)24 annealing was undertaken in the absence of dNTPs, which were subsequently added to the first strand master mix.

### SMART^{™} 1st strand cDNA syntheses

The first and the second strand synthesis are performed in the same tube that enhances the synthesis procedure and maximizes recovery of cDNA. The method described here uses PowerScript™ Reverse Transcriptase (BD Biosciences Clontech), a point mutant of Moloney murine leukemia virus (MMLV) reverse transcriptase. PowerScript™ lacks RNase H activity, but retains wild-type polymerase activity, so longer cDNA fragments can be synthesized than wild-type MMLV RT.

The recommended SMART^{™} protocol (BD Biosciences Clontech) was modified by including 1 mM dNTP's in the initial step in the procedure where the RNA is heated to 70°C for 2 min. We show here that this modification increased the yield of cRNA, from defined amounts of starting Total RNA (see Table 2 below). This modification allows a reduced number of PCR cycles with limited starting material, and should help minimize any distortions to the mRNA distribution.

cRNAs synthesised from 50ng or 5ng total RNA. 1mM dNTPs were added at the RNA-primer annealing step plus the cDNA synthesis mix [dNTPs in steps 1 and 2], or only in the cDNA synthesis mix [dNTPs in step 2 only]. The numbers of PCR cycles required to subsequently generate - 20µg of cRNA are shown in Table 2. Primer mix was prepared by mixing equal volumes of 1µM SMART™-T7-oligo(dT)30VN oligonucleotide SEQ ID 1 and 1µM SMART^{™} II A oligonucleotide SEQ ID 2 (BD Biosciences Clontech) so that both were 0.5µM. We have used a reduced concentration of both primers [Baugh, et al (2001)] reported that reduced concentrations of oligo dT primer reduced non-specific artifacts when using lower amounts of template RNA, and we reasoned that reduced concentrations of the novel primer SMART™-T7-oligo(dT)30VN oligonucleotide SEQ ID 1 and of SMART^{™} II A oligonucleotide SEQ ID 2 would be required as we are starting with much less total RNA than required in the standard SMART^{™} protocol.

3µl of Total RNA were mixed with 2µl of 0.5µM primer mix. RNA / primer was heated at 70°C for 2 min, then at 4°C for 5 min in a thermal cycler.

At 4°C, 5µl of First Strand Mastermix was added to each reaction. First Strand Mastermix was prepared from components of SMART^{™} PCR cDNA Synthesis Kit (BD Biosciences Clontech, # K1052-1) and 2ul 5x First-strand buffer (250mM Tris-HCl, pH8.3, 30mM magnesium chloride and 375mM potassium chloride), 1ul 20mM dithiothreitol (DTT), 1ul 10mM dNTPs (10mM each dATP, dCTP, dGTP, dTTP), 1ul PowerScript™ Reverse Transcriptase was added. Reactions were mixed by gentle pipetting, then heated for 1h at 42°C, then 4°C for 5 min in a thermal cycler.

### PCR Amplification of SMART^{™} synthesised cDNA

The method described here uses Advantage® 2 Polymerase Mix (BD Biosciences Clontech), which allows efficient and accurate amplification of cDNA templates by long-distance PCR [Barnes, 1994]. AdvantageTM 2 products are described in US 5,436,149.

The Advantage® 2 Polymerase Mix contains TITANIUM^{™} Taq DNA Polymerase, a nuclease-deficient N-terminal deletion of Taq DNA Polymerase, and a minor amount of a proofreading polymerase. Advantage® 2 Polymerase Mix also contains TaqStart™ Antibody (BD Biosciences Clontech) to provide automatic hot-start PCR (Kellogg et al., 1994) and reduce non-specific priming of template. This combination allows efficient amplification of full-length cDNAs with a significantly lower error rate than that of conventional PCR (Barnes, 1994).

90µl PCR Master Mix was added to each cDNA synthesis using the same tube as for SMART^{™} cDNA synthesis. 5' PCR IIA primers SEQ ID 3 was identical sequence to that used in the BD Biosciences Clontech SMART^{™} cDNAsynthesis kit (Cat # K1052-1). This primer will bind to both ends of the SMART^{™} cDNA and allow PCR amplification. PCR was set up using components form Advantage® 2 polymerase Kit (BD Biosciences Clontech, Cat# 8430-1) with 10ul 10X Advantage® 2 PCR Buffer (40mM Tricine-KOH, pH9.2, 15mM potassium acetate, 3.5 mM magnesium acetate), 2ul 50X dNTP Mix (10 mM of each dNTP), 2ul 10uM 5' PCR Primer II A SEQ ID 3, and 2ul 50X Advantage® 2 Polymerase Mix and 74ul water.

Thermal cycling parameters of the PCR reactions were 60 sec at 95°C for hot start, followed by 3 step cycling for a pre-determined numbers of cycles (eg 11 cycles for 50ng starting total RNA or 15 cycles for 5ng total RNA) of denaturation for 15 sec at 95°C, annealing for 30 sec at 65°C, and extension for 6 min at 68°C. Reactions were then held at 4°C in the thermal cycler until purification. 50ng reactions were typically cycled for 10 or 11 cycles, and 5ng reactions for 14 or 15 cycles.

We have devised an improved method for determining the optimal number of PCR cycles that are required from a given amount of starting material. For any PCR-based method one of the key factors is to perform the minimum numbers of PCR cycles (for a given amount of starting material) that will result in sufficient target for the intended downstream application. If too many cycles are performed, then it is likely that the representation of mRNAs could be biased as the PCR reactions are unlikely to be identical for each RNA template in the complex mixture, and PCR may also reach a plateau. If too few cycles are performed then insufficient PCR product is obtained for subsequent applications. BD Biosciences Clontech SMART^{™} methodology recommends an optimisation strategy that involves examination of the amplified PCR products generated after different numbers of cycles on agarose gel electrophoresis and then visual estimation of the optimal amplification. In the method described here, we determine empirically for a given amount of starting total RNA (eg 50ng or 5ng), the minimum numbers of PCR cycles that are required such that when the entire PCR products are then used for *in-vitro* transcription (IVT) reactions to generate cRNA, we obtain sufficient cRNA for the intended downstream application. This can be done easily by starting with a given amount of total RNA (eg 5ng or 50ng) by setting up identical reactions and performing PCR for defined numbers of cycles (or by removing aliquots form a PCR reaction for analysis and performing additional cycles on the remainder of the PCR reaction). The entire PCR products from each reaction (for example 50ng starting total RNA that has undergone PCR thermal cycling for 9,10,11,12,13 or 14 cycles) is purified and used for IVT. The number of PCR cycles that gives the minimum amount of cRNA that is sufficient for the downstream application is then determined. This number of cycles can then be used routinely for similar studies with other RNA's at the same starting concentrations.

For example, in this study reported here we describe downstream use on Affymetrix GeneChip® probe arrays. If the PCR product was to be used for another downstream application then an identical strategy could be used. The cycling parameters in this protocol have been optimised using a MJ Research PTC200 thermal cycler. However, the numbers of cycles required for different amounts of starting total RNA should be determined empirically in initial experiments as optimal parameters may vary with different templates, thermal cyclers, or if different enzymes are used for cDNA synthesis or PCR. Once the optimal conditions are determined for a specific amount of starting total RNA, then subsequent experiments with this amount of RNA under identical conditions should results in similar yields of product.

This PCR step can also be enhanced by addition of dNTP's in the initial step in the procedure where the RNA is heated to 70oC for 2 minutes (see above for details). This means that a reduced number of PCR cycles are required with limited amounts of starting material and therefore help mimimise any distortions to the mRNA distributions.

PCR products were purified using QIAGEN Qiaquick columns as per manufacturer's instructions. For the elution: 30µl of elution buffer was applied to column and left to stand for 2min prior to centrifugation. Eluted volumes were adjusted to 30µl with molecular biology grade water. PCR purification could be performed using other PCR purification methods or commercial kits.

### Synthesis of Biotin-Labelled cRNA (IVT) from amplified cDNAs

ENZO ® BioArray™ HighYield™ RNA Transcript Labeling Kit, Affymetrix 900182 was used for IVT, but this could be obtained from a number of other suppliers. To each 30µl of purified PCR was added 20µl of Mastermix. [5µl 10x HY Reaction Buffer, 5µl 10x Biotin Labelled Ribonucleotides, 4µl 10x DDT, 4µl 10x RNase Inhibitor Mix, 2µl 20x T7 RNA Polymerase]. IVT reactions were incubated at 37°C for 5hrs using thin-walled 0.2ml PCR tubes.

50ul water was added per sample and then the entire reactions were purified using RNeasy® Mini columns (QIAGEN) as per manufacturer's instructions. Purified RNA was eluted with 2 sequential applications of 50µl RNase-free water i.e. 100µl final volume.

5ul eluted RNA was added to 95ul water per well of a 96-well UV plate (Costar # 3635) and used for A260 measurement (Molecular Devices, Spectramax) to quantitate the amount of RNA.

### Fragmentation and Hybridization

Followed the instructions described in the Affymetrix GeneChip® Expression Analysis Technical Manual.

### Data Analysis

Data were analysed for a number of assay performance criteria, in order to evaluate the effectiveness of the novel amplification protocol for Affymetrix GeneChip® array expression analysis. These criteria were labelled cRNA yield; Standard array quality metrics including Raw Q, Background, Scaling Factor, Percent Present Calls, and 3' and 5' Signal Intensity ratio of control probe sets; Linearity and sensitivity of amplification as quantified using spike-in bacterial poly-A controls; Reproducibility; Concordance analysis of differential gene expression between the standard Affymetrix protocol and the novel amplification protocol; Confirmation of gene expression changes by an independent technique (RT-PCR).

### Labelled cRNA Yield

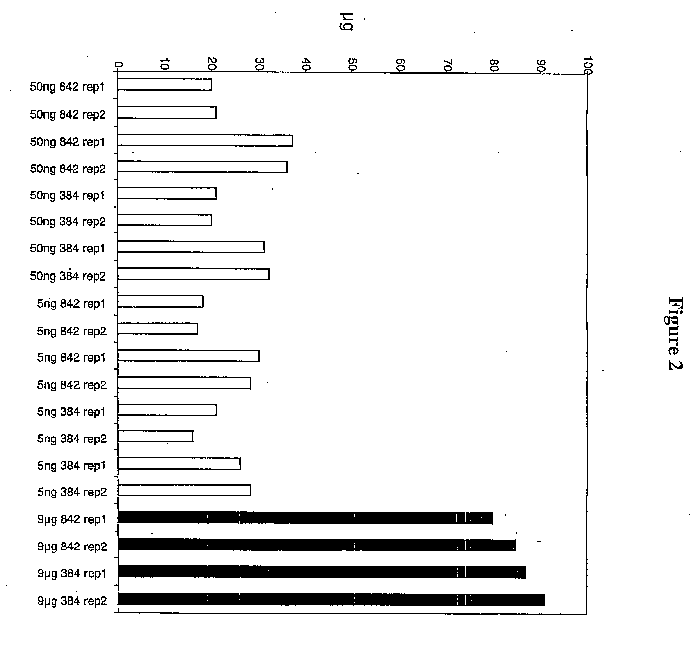

The yield of labelled cRNA is critical because sufficient target needs to be generated for downstream applications for example Affymetrix GeneChip® probe arrays. The required amount of labeled cRNA for Affymetrix GeneChip® is 10-15 µg for each genome array. A series of experiments were carried out using different amounts of starting material. Total RNAs were extracted from a T3 Grade 3 (sample 384) and a superficial Ta Grade 1 (sample 842) transitional cell bladder carcinoma and used as template for preparing probes. Replicate probes were prepared from 9µg total RNA using the conventional Affymetrix protocol and from 50ng and 5ng total RNA using the novel amplification method described here. Table 3 below gives a summary of the probes synthesised, the number of thermocycles used and their respective cRNA yields, and indicates which probes were used for subsequent fragmentation and hybridization to Affymetrix GeneChips. Note that for the conventional probe syntheses oligo dT annealing was undertaken without the presence of dNTPs. The quantity of cRNA obtained was measured by absorbance at 260 nanometers (nm) after purification and are plotted (Figure 2) to demonstrate the repeatability of amplification reactions. As shown in Figure 2, the quantities of the labeled cRNA obtained from samples of 5ng or 50 ng total RNA with the novel amplification protocol were comparable with the range anticipated from the standard protocol.

### Standard GeneChip® Array Quality Metrics

To further evaluate whether the novel amplification protocol is suitable for preparing targets for GeneChip® array expression analysis, 10 µg of cRNA targets, generated from the experiments described previously, were hybridized on the GeneChip® Human Genome U133A (HG-U133A) arrays under standard conditions and washed using the EukGE-WS2v4 fluidics protocol. After scanning, the chips were subjected to a visual QC check for excessive background and the presence of staining artefacts. The data were then analyzed using Affymetrix Microarray Suite 5.0 (MAS 5.0) software and various quality control metrics were obtained.

Table 3 gives a summary of the QC metrics taken from the .RPT files. The scaling factors were consistent for all chip hybridisations with no extreme values observed.

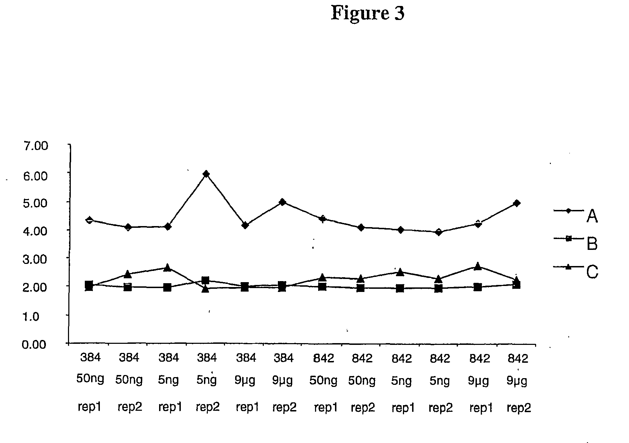

Raw Q, Background, and Scaling Factor values were examined to evaluate the overall sample quality with the targets prepared according to the two protocols (novel amplification protocol and the standard protocol) (see Fig 3). As shown in Table 4, comparable values were obtained for all three parameters. For example, the Background values were all about 100, as anticipated for typical experiments. The Scaling Factors were also within threefold range-even when comparing the data from 5 ng of starting material amplified with the novel amplification protocol.

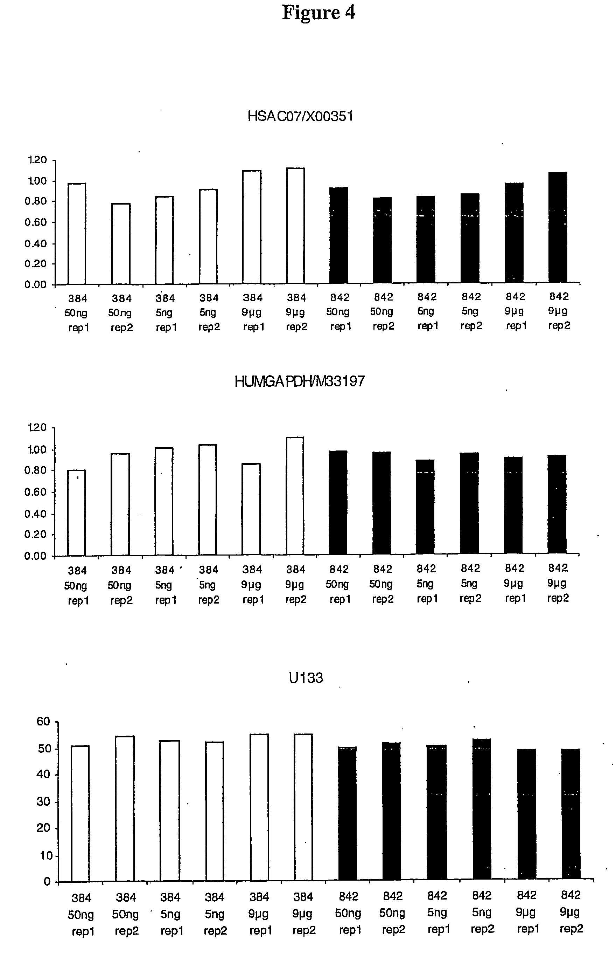

Affymetrix GeneChip® arrays are designed predominantly with probes selected adjacent to the poly-A tail of the mRNA. This design strategy along with the inherent generation of shorter fragment from additional amplifications may create targets that are skewed to the 3' end. To examine this phenomenon, Affymetrix have created probe sets for specific maintenance genes (e.g., GAPDH, actin), and these probe sets are designed to the 3', middle, and 5' regions of the transcript. The 3' probe set Signal Intensity can then be compared to the 5' probe set Signal Intensity (3'/5' ratio) to evaluate the efficiency of the transcription reaction. As shown in Fig 4 (top and middle), the 5 ng and 50ng total RNA samples with the novel amplification protocol described here, produced the 3'/5' ratio for GAPDH and Actin genes, of approximately 1, which are equivalent to those samples processed with the standard protocol and well within the Affymetrix recommended range of 3. Actin transcript represented on the array was longer than the GAPDH gene, with 1,761 bases (with the 5' probe set within 1178-1712 bases, and the Middle probe set within 589-1117 bases), but the 3'/5' ratios were still maintained. We also calculated the 3'/Middle probe set ratio (3'/M) of the GAPDH and Actin, genes (data not shown) which was also very similar to those samples processed with the standard protocol.

The Percent Present Calls comparison was used to globally assess the data representations. As shown in Fig 4 (bottom), comparable Percent Present Call values were obtained with reducing amounts of starting materials. Even at 5 ng of starting total RNA, about 50 percent of the probe sets were still called as Present by the Affymetrix MAS5.0 software algorithm.

### Linearity and Sensitivity

The ability of any amplification protocol to accurately detect differences in expression levels is highly dependent on the assay's linearity and sensitivity.

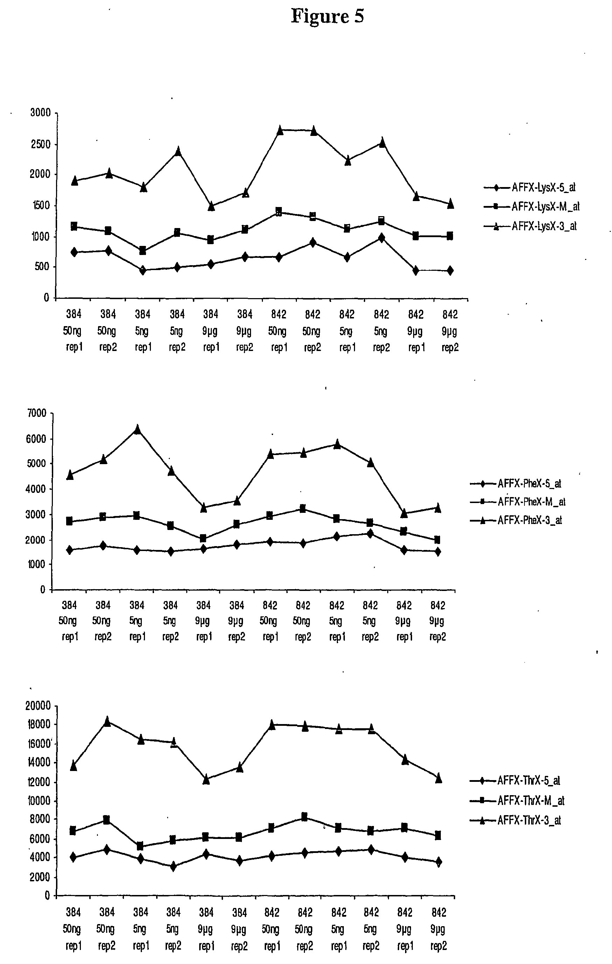

The novel amplification protocol was evaluated for both parameters by analyzing the spike-in poly-A control transcripts in a complex sample. 4 spike-in control transcripts were spiked into the complex human bladder biopsy total RNA samples at various concentrations. 3 were in *vitro*-generated bacterial poly-A controls - *lys, phe,* and thr and the other spike was a commercially available poly-A control RNA for kanamycin. *Lys, phe,* and thr are represented by probe sets on the U133A GeneChips and so these spikes can be detected when added to RNA samples for profiling. Kanamycin spike-in poly-A control transcript can be measured by a specific real-time PCR based TaqMan assay. 5 ng and 50 ng of total RNA were labelled with the novel amplification protocol, the target was hybridized on HG-U133A arrays, and the Signal Intensities for the controls were plotted (Figure 5), and the three transcripts were detected by all the probes regardless of the method.

### Reproducibility of replicates

Reproducibility is a key requirement for any amplification protocol, and it is essential for generating reliable results. Two independent target preparations with 5 ng and 50 ng of two different total RNAs (RNA IDs 384 and 842) using the novel amplification protocol were hybridized to HG-U133A arrays.

Pearson correlation values (Tables 5 and 6) were calculated for the amplified and non-amplified probes derived from each sample. The Pearson correlation coefficients were all >0.95 thus indicating very good agreement between each set of replicates. However, as the standard and novel amplification protocol use different total RNA amounts and different protocols, it is not recommended that results obtained from amplified and non-amplified samples are directly compared.

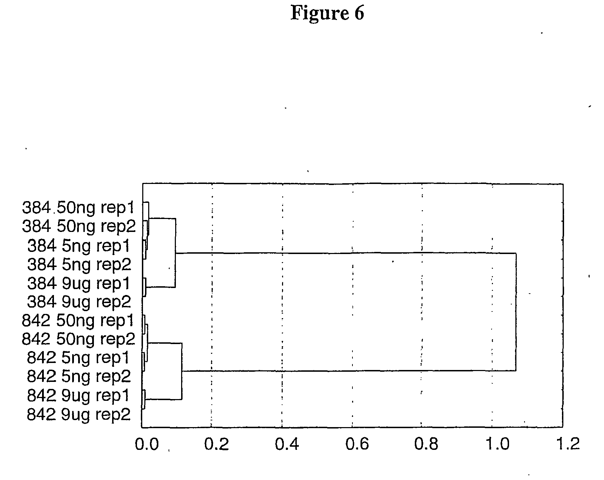

Data sets for each chip hybridisation were clustered using Statistica software (Tulsa, USA): the tree diagram (Figure 6) prepared using Ward's method shows that the two samples were clustered separately and that, as expected, within each sample the amplified probes were distinguishable from the non-amplified probes

### Scatter plots of the replicates

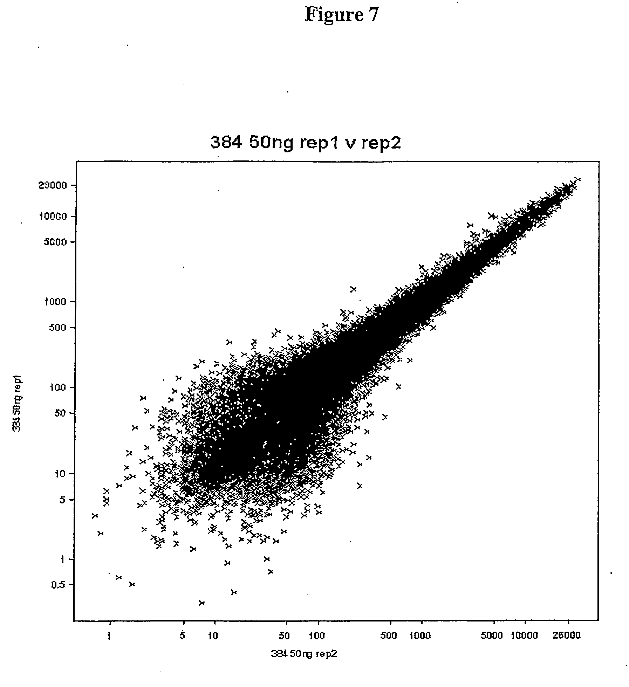

Log scale scatter plots for replicate samples indicate typical profiles about the xy diagonal which was indicative of good reproducibility for the replicates. A representative log scale scatter plot for 384 50ng rep1 vs rep2 (Figure 7) illustrates the typical profile. Most of the scatter was noticeable at signal values <500; this is due to mainly to the limitations of the technology platform and is independent of probe synthesis method.

### Data analysis

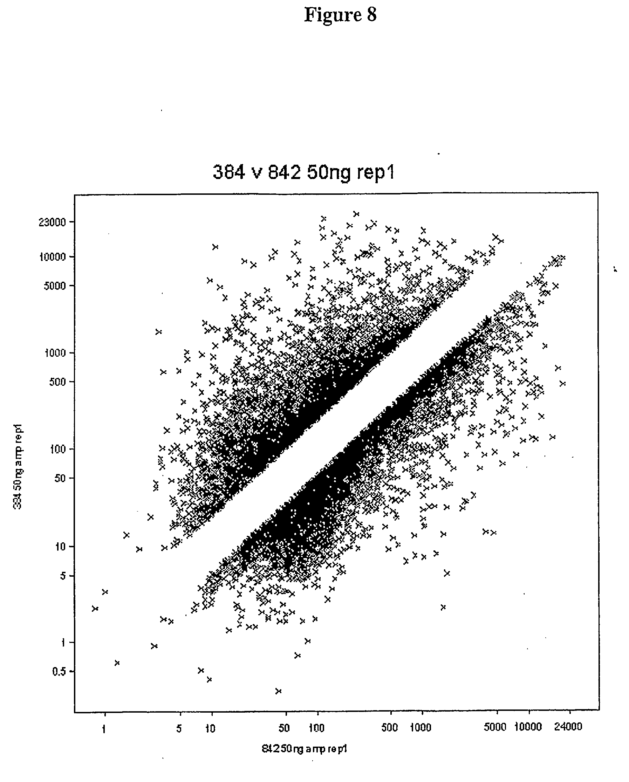

The numbers of genes changing by more than 2 fold in any direction were identified using Spotfire software (Goteborg, Sweden) and summarised in Tables 7 and 8. Log scale scatter plots indicate genes changing >2 fold (equivalent to > |log₁₀ 0.3|). Figure 8 shows a log scale scatter plot for 384 v 842 50ng rep1, which is typical of the scatter plots for all other comparisons.

We have compared scatter plots of all the log ratio sets of all the pairwise comparisons of non-amplified versus amplified samples to obtain a measure of relative gene expression for the samples being compared. Figure 9 shows the log ratio Set A compared to Set D. All log ratio sets, when compared to Set A, show a similar distribution along the xy diagonal. As a qualitative observation, it is reasonable to assume that the ratios sets are all alike - indeed the ratios derived from the novel amplification method are no more different than the Set A versus Set B comparison where all data was obtained using standard protocol. The Pearson correlation coefficients vary from 0.81 to 0.87 with the highest figure reported for the comparison between non-amplified probes and the lowest figure of 0.81 reported for Set A versus Set F. - these are all good correlation values for log ratio comparisons. Note that the data, including that presented in Figure 9, were filtered to remove noise i.e. for any given gene where signals were <100 across all twelve GeneChip® hybridisations. Table 4 shows that the average background signal reported for this experiment was 110 ± 22 SD.

Table 6 shows that there were similar numbers of genes changing >2 fold for each comparison between samples 384 and 842 (average = 7844 ± 444 SD) regardless of the probe synthesis method. In order to determine how many of these changes are for the same genes in each set, Set A was used as the reference because these data were obtained using standard Affymetrix protocol. However, by taking the comparison of Set A versus all other sets (Table 6) it can be seen that the number of genes in common is less than the figure of 7844 reported above, and that this includes Set A versus Set B where all data were obtained using standard Affymetrix protocol. Furthermore, it can be seen that there were only 2385 genes in common across all five comparisons to Set A. This result is at least in part due to the limitation of setting an arbitrary cut off value (in this case >2 fold).

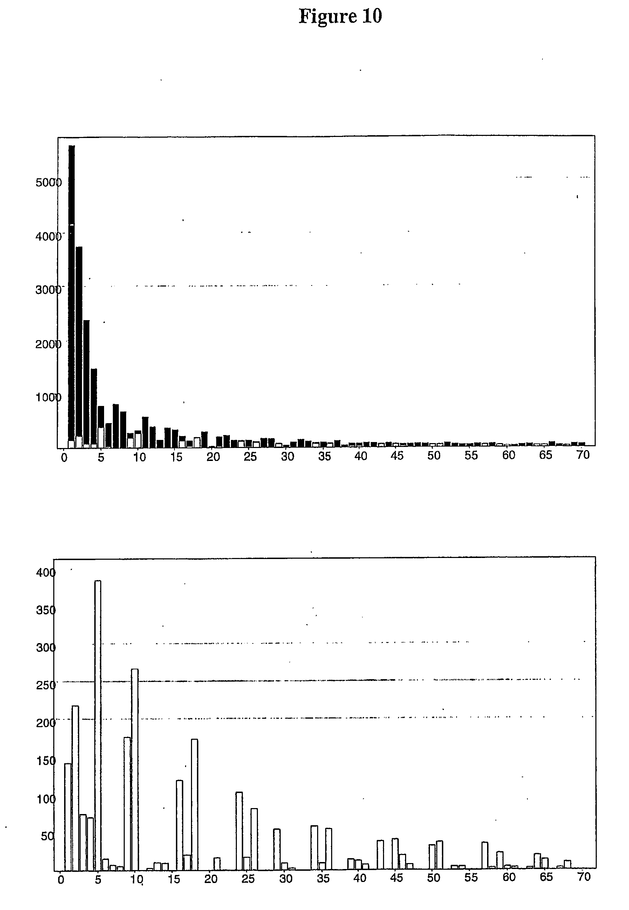

k-means clustering was employed on the data sets using Spotfire software with default settings and arbitrarily chosen to calculate 70 clusters. The data sets were left intact with no background signal filtering employed. The frequency histograms (Figure 10) depict the numbers of genes in each cluster and whether they were changing >2 fold in which shown in outline (white). Note that the largest cluster contains 5488 genes, and that the first four clusters comprise approximately 60% of all genes. Collectively the clusters in the lower ) histogram represent the 2385 genes changing >2 fold in common - see Table 7.

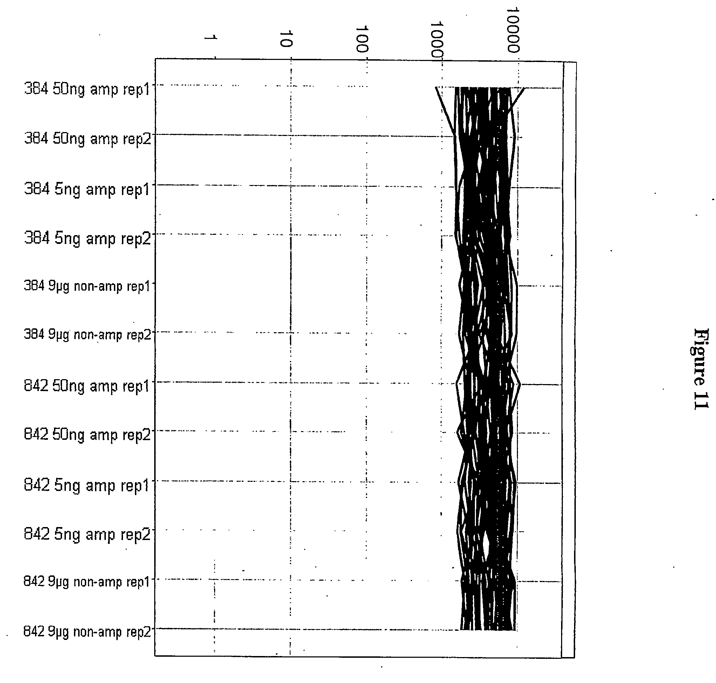

Attention will now be paid to selected representative clusters so as to illustrate that the gene transcript profiles can be accurately captured using the CPA method. Firstly, some pointers to help with analysis of the clusters presented in Figures 11, 12 and 13.
- sample names are labelled on the x axis
- y axis of each cluster is log₁₀ scale

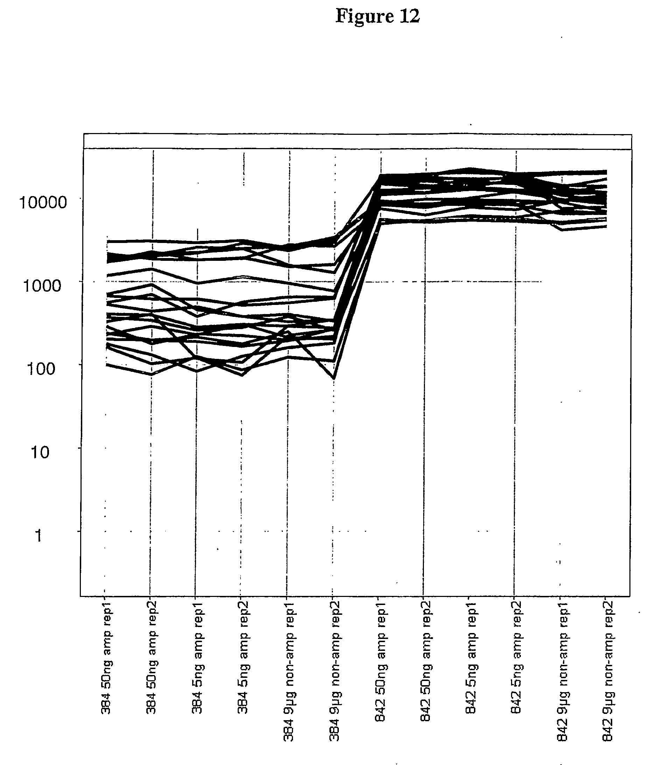

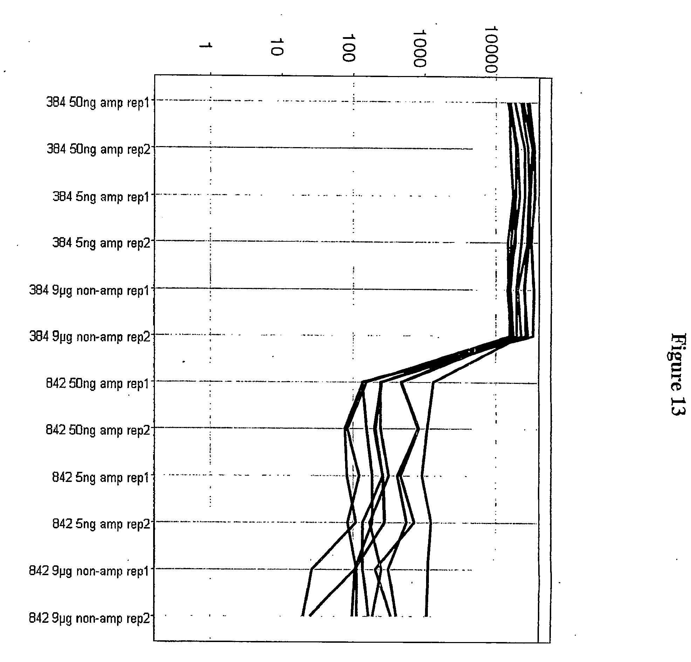

Looking at representative clusters in detail, Figure 11 shows a cluster demonstrating no significant differential gene expression between the T3 grade 3 (sample 384) and a superficial Ta grade 1 (sample 842) tumours since the individual gene profiles appear to be horizontal across all samples. However when differential gene expression does occur, as exemplified by the clusters depicted in Figures 12 and 13, it can be seen that the amplified and non-amplified probes are generating equivalent data and that there is no observable bias.

As stated previously the first four clusters comprise about 60% of the genes on the GeneChip® and in the case of cluster 1 all the genes demonstrate very low signal magnitudes (∼<100). This is very close to the detection threshold of the Affymetrix technology and signals in this region may or may not be due to real gene expression regardless of the method of probe synthesis. Upon inspection of all the clusters in the data set it is apparent that differential expression is represented accurately by expression profiles derived using the novel amplification protocol and as well as the Affymetrix standard protocol.

It is this qualitative analysis, along with the scatter plots of log ratios and the QC metrics, which demonstrate that the novel amplification protocol can generate transcript profiles from 5-50ng total RNA with acceptable maintenance of profile integrity.

Several gene changes have been confirmed by RT-PCR using the same Total RNAs (384 and 842) and arc concordant with Affymetrix GeneChip® results (data not shown).

### Conclusions

We found that the novel amplification protocol is suitable for robustly amplifying and labelling as low as 5 ng of total RNA for expression profiling. The assay demonstrated good cRNA yield, sensitivity, and reproducibility. In the work exemplified here we have focused downstream application of the cRNA, that is generated via the novel amplification method, for Affymetrix GeneChip® probe arrays. However, the cRNA could be used for other downstream applications, including other gene expression profiling platforms. The results closely approximate the standard Affymetrix method thus maintaining the integrity of the transcript profile and the QC metrics are comparable to those obtained using standard protocol. The protocol provides scope for further improvement in particular the number of thermocycles could be reduced further still so that the final yield of biotinylated cRNA is approximately 10µg since this is the minimum quantity actually required for hybridisation to an Affymetrix GeneChip®.

As described here, there is also scope to use lower amounts of total RNA.

**Table 1**

| Sample | Addition of dNTPs during annealing | Method |
|---|---|---|
| 384 50ng amp rep1 | yes | 15 cycles PCR + IVT |
| 384 50ng amp rep2 | yes | 15 cycles PCR + IVT |
| 384 5ng amp rep1 | yes | 11 cycles PCR + IVT |
| 384 5ng amp rep2 | yes | 11 cycles PCR + IVT |
| 384 9µg non-amp rep1 | no | IVT only |
| 384 9µg non-amp rep2 | no | IVT only |
| 842 50ng amp rep1 | yes | 15 cycles PCR + IVT |
| 842 50ng amp rep2 | yes | 15 cycles PCR + IVT |
| 842 5ng amp rep1 | yes | 11 cycles PCR + IVT |
| 842 5ng amp rep2 | yes | 11 cycles PCR + IVT |
| 842 9µg non-amp rep1 | no | IVT only |
| 842 9µg non-amp rep2 | no | IVT only |

**Table 2**

| The effect of dNTP addition at the primer-annealing step on yield of cRNA | | |
|---|---|---|
| Starting Total RNA | dNTPs in step 2 only | dNTPs in steps 1 and 2 |
| 50ng Total RNA | 12 | 10 |
| 5ng Total RNA | 16 | 14 |

**Table 3**

| Probe syntheses cRNA yield and number of thermocycles used. | | | | | |
|---|---|---|---|---|---|
| **No.** | **sample** | **PCR cycles** | **IV T** | **Yield cRNA (µg)** | |
| 1 | 50ng 842 rep1 | 10 | Yes | 20 | Not used |
| 2 | 50ng 842 rep2 | 10 | " | 21 | " |
| 3 | 50ng 842 rep1 | 11 | " | 37 | Used for fragmentation + hyb |
| 4 | 50ng 842 rep2 | 11 | " | 36 | " |
| 5 | 50ng 384 rep1 | 10 | " | 21 | Not used |
| 6 | 50ng 384 rep2 | 10 | " | 20 | " |
| 7 | 50ng 384 rep1 | 11 | " | 31 | Used for fragmentation + hyb |
| 8 | 50ng 384 rep2 | 11 | " | 32 | " |
| 9 | 5ng 842 rep1 | 14 | " | 18 | Not used |
| 10 | 5ng 842 rep2 | 14 | " | 17 | " |
| 11 | 5ng 842 rep1 | 15 | " | 30 | Used for fragmentation + hyb |
| 12 | 5ng 842 rep2 | 15 | " | 28 | " |
| 13 | 5ng 384 rep1 | 14 | " | 21 | Not used |
| 14 | 5ng 384 rep2 | 14 | " | 16 | " |
| 15 | 5ng 384 rep1 | 15 | " | 26 | Used for fragmentation + hyb |
| 16 | 5ng 384 rep2 | 15 | " | 28 | " |
| 17 | 842 9µg rep1 | 0 | " | 80 | " |
| 18 | 842 9µg rep2 | 0 | " | 85 | " |
| 19 | 384 9µg rep1 | 0 | " | 87 | " |
| 20 | 384 9µg rep2 | 0 | " | 91 | " |

**Table 4**

| **Array Quality Metrics Comparisons** | | | | | |
|---|---|---|---|---|---|
| **sample** | **Raw Q** | ***Background** | **Scaling Factor** | **gapdh 3'/5'** | **actin 3'/5'** |
| 384 50ng amp rep1 | 4.32 | 115.14 | 1.96 | 0.80 | 0.97 |
| 384 50ng amp rep2 | 4.06 | 97.83 | 2.43 | 0.96 | 0.78 |
| 384 5ng amp rep1 | 4.10 | 92.74 | 2.66 | 1.01 | 0.84 |
| 384 5ng amp rep2 | 5.99 | 172.10 | 1.94 | 1.03 | 0.91 |
| 384 9µg non-amp rep1 | 4.16 | 99.42 | 1.99 | 0.86 | 1.08 |
| 384 9µg non-amp rep2 | 5.00 | 116.93 | 2.00 | 1.10 | 1.11 |
| 842 50ng amp rep1 | 4.41 | 108.02 | 2.35 | 0.97 | 0.92 |
| 842 50ng amp rep2 | 4.13 | 98.75 | 2.30 | 0.96 | 0.82 |
| 842 5ng amp rep1 | 4.04 | 98.89 | 2.57 | 0.88 | 0.83 |
| 842 5ng amp rep2 | 3.94 | 94.44 | 2.30 | 0.94 | 0.86 |
| 842 9µg non-amp rep1 | 4.26 | 99.84 | 2.75 | 0.91 | 0.96 |
| 842 9µg non-amp rep2 | 5.00 | 129.86 | 2.25 | 0.92 | 1.06 |

| | | | | | |
|---|---|---|---|---|---|
| *Average Background = 110 ± 22 SD | | | | | |

The targets generated from the standard protocol or the novel amplification protocol were hybridised to U133A Human Genome Genechips under standard conditions and washed using the EukGE-WS2v4 fluidics protocol. Table 2 gives a summary of the QC metrics taken from the Affymetrix MAS5.0 .rpt files. All of the metrics are within the acceptable range of values.

**Table 5**

| Pearson correlation matrix for the biopsy "842" samples compared against each other. | | | | | | |
|---|---|---|---|---|---|---|
| **Sample 842** | 50ng amp r1 | 50ng amp r2 | 5ng amp r1 | 5ng amp r2 | 9µg non-amp r1 | 9µg non-amp r2 |
| 50ng amp r1 | 1.00 | | | | | |
| 50ng amp r2 | 0.99 | 1.00 | | | | |
| 5ng amp r1 | 0.99 | 0.99 | 1.00 | | | |
| 5ng amp r2 | 0.99 | 0.99 | 0.99 | 1.00 | | |
| 9µg non-amp r1 | 0.95 | 0.95 | 0.95 | 0.95 | 1.00 | |
| 9µg non-amp r2 | 0.96 | 0.95 | 0.95 | 0.95 | 0.99 | 1.00 |

**Table 6**

| Pearson correlation matrix for the biopsy "384" samples compared against each other. | | | | | | |
|---|---|---|---|---|---|---|
| **Sample 384** | 50ng amp r1 | 50ng amp r2 | 5ng amp r1 | 5ng amp r2 | 9µg non-amp r1 | 9µg non-amp r2 |
| 50ng amp r1 | 1.00 | | | | | |
| 50ng amp r2 | 0.98 | 1.00 | | | | |
| 5ng amp r1 | 0.98 | 0.99 | 1.00 | | | |
| 5ng amp r2 | 0.98 | 0.99 | 0.99 | 1.00 | | |
| 9µg non-amp r1 | 0.96 | 0.96 | 0.96 | 0.95 | 1.00 | |
| 9µg non-amp r2 | 0.96 | 0.96 | 0.96 | 0.96 | 0.99 | 1.00 |

**Table 7**

| Genes changing >2 fold for each set of replicates. | | |
|---|---|---|
| **Set** | **Comparison** | **Genes changing > 2 fold** |
| A | 9µg 384 non amp rep1 versus 9µg 842 non amp rep1 | 7440 |
| B | 9µg 384 non amp rep2 versus 9µg 842 non amp rep2 | 7477 |
| C | 50ng 384 amp rep1 versus 50ng 842 amp rep1 | 7620 |
| D | 50ng 384 amp rep2 versus 50ng 842 amp rep2 | 7744 |
| E | 5ng 384 amp rep1 versus 5ng 842 amp rep1 | 8508 |
| F | 5ng 384 amp rep2 versus 5ng 842 amp rep2 | 8275 |

**Table 8**

| Intersection of sets demonstrating number of gene expression changes in common for each pairwise comparison | | |
|---|---|---|
| **Set comparisons** | **> 2 fold common to both sets** | **Common to all 5 comparisons** |
| A and B | 4737 | |
| A and C | 4661 | |
| A and D | 4738 | 2385 |
| A and E | 4803 | |
| A and F | 4678 | |

### SEQUENCES

SEQ ID No 1
   5'AAGCAGTGGTATCAACGCAGAGTGGCCAGTGAATTGTAATACGACTCACTATA GGGAGGCGG(T)₃₀VN-3'
   where V is A, G , or C and N is any base
SEQ ID No. 2 (described in US 5,962,271 and US 5,962,272)
   5'-AAGCAGTGGTATCAACGCAGAGTACGCGGG-3'
SEO ID No. 3 (described in US 5,962,271 and US 5,962,272)
   5'-AAGCAGTGGTATCAACGCAGAGT-3'
SEQ ID No 4
   5'AAGCAGTGGTATCAACGCAGAGTAATACGACTCACTATAGGGAGA(T)₂₄VN-3'
   wherein V is A, G , or C and N is any base.
SEQ ID No. 5
   5' GCATTAACCCTCACTAAC 3'
SEQ ID No. 6
   5' TAATACGACTCACTATA 3'
SEQ ID No. 7
   5' AATACGACTCACTATAGGGAGA 3'
SEQ ID NO. 8
   5' GGCCAGTGAATTGTAATACGACTCACTATAGGGAGGCGG 3'
SEQ ID No. 9
   5' ATTTAGGTGACACTATA 3'

### REFERENCES

Assersohn, L, Gangi, L, Zhao, Y, Dowsett, M, Simon, R, Powles, TJ, Liu, ET (2002) The feasibility of using fine needle aspiration from primary breast cancers for cDNA microarray analyses. Clin Cancer Res ,8:794-801
Van Gelder, RN, von Zastrow, ME, Yoo, 1 A, Dement, WC, Barchas, JD, Eberwine, JH (1990) Amplified RNA synthesized from limited quantities of heterogeneous cDNA. Proc Natl Acad Sci USA, 87, 1663-1667
Eberwine, J, Yeh, H, Miyashiro, K, Cao, Y, Nair, S, Finnell, R, Zettel, M, Coleman, P (1992) Analysis of gene expression in single live neurons. Proc Natl Acad Sci USA 89, 3010-3014
Phillips, J, and Eberwine, JH (1996) Antisense RNA amplification:a linear amplification method for analyzing the mRNA population from single living cells. Methods 10:283-288
Affymetrix GeneChip® Expression Analysis Technical Manual available at http://www.affymetrix.com.
Mahadevappa, M, & Warrington, J (1999) A high-density probe array sample preparation method using 10-to 100-fold fewer cells. Nat Biotechnol 17, 1134-1136
Pabón, C, Modrusan, Z, Ruvolo, MV, Coleman, IM, Daniel, S, Yue, H, Arnold, LJ Jr, Reynolds, MA (2001) Optimized T7 amplification system for microarray analysis. Biotechniques 31, 874-879
Luo, L, Salunga, RC, Guo, H, Bittner, A, Joy, KC, Galindo, JE, Xiao, H, Rogers, KE, Wan, JS, Jackson, MR (1999) Gene expression profiles of laser-captured adjacent neuronal subtypes. Nat Med 5, 117-122
Ohyama, H,Zhang, X, Kohno Y, Alevizos, I, Posner M, Wong DT, Todd R: (2000) Laser capture microdissection-generated target sample for high-density oligonucleotide array hybridization. Biotechniques, 29, 530-536
Affymetrix Technical Note GeneChip ® Eukaryotic Small sample Target Labeling Assay Version II [http://www.affymetrix.com/Download/manuals]
Luzzi, V, Holtschlag, V, Watson, MA (2001) Expression Profiling of ductal carcinoma in situ by laser capture microdissection and high-density oligonucleotide arrays. Am. J. Pathology, 158, 2005-2010
Wang E, Miller LD, Ohnmacht, GA, Liu ET, Marincola, FM (2000) High-fidelity mRNA amplification for gene profiling. Nat. Biotechnol. 18, 457-459
Hu L, Wang J, Baggerly K, Wang H, Fuller GN, Hamilton SR, Coombes KR, Zhang W (2002) Obtaining reliable information from minute amounts of RNA using cDNA microarrays. BMG Genomics, 3, 16
Akowitz, A. and Manuelidis, L. (1989) A novel cDNA/PCR strategy for efficient cloning of small amounts of undefined RNA. Gene 81, 295-306
Belyavsky, A., Vinogradova, T. and Rajewsky, K. (1989) PCR-based cDNA library construction : general cDNA libraries at the level of a few cells Nucleic Acids Res. 17, 2919-2932
Domec, C, Garbay, B., Fournier, M. Bonnet, J. (1990) cDNA library construction form small amounts of unfractionated RNA : association of cDNA synthesis with polymerase chain reaction amplification. Anal Biochem. 188, 422-426
Brady, G., Barbara, M., Iscove, N., (1990). Representative in vitro cDNA amplification from individual hematopoietic cells and colonies. Methods Mol. Cell. Biol. 2, 17-25
Apte, A.N., and Siebert, P.D. (1993) Anchor-ligated cDNA libraries : a technique for generating a cDNA library for the immediate cloning of the 5'ends of mRNAs. Biotechniques, 15, 890-893
Fromont-Racine, M.A., Bertrand, R., Pictet, R., and Grange, T. (1993). A highly sensitive method for mapping the 5' termini of mRNAs. Nucleic Acids Res. 21, 1683-1684
Kato, S, Sekine, S., Oh, S.W., Kim, N.S., Umezawa, Abe, N., Yokoyama-Kobayashi, M. and Aoki, T. (1994) Construction of a human full-length cDNA bank. Gene 150, 243-250
Maruyama, K. Sugano, S. (1994) Oligo-capping : a simple method to replace the cap structure of eukaryotic mRNAs with oligonucleotide. Gene 138, 171-174
Frohman, M.A., Dush, M.K. and Martin, G.R. (1988) Rapid production of full-length cDNAs from rare transcripts : amplification using a single gene-specific oligonucleotide primer. Proc. Natl. Acad. Sci. USA 85, 8998-9002]
Chenchik, A., Zhu, Y. Y., Diatchenko, L., Li, R., Hill, J. & Siebert, P. D. (1998) Generation and use of high-quality cDNA from small amounts of total RNA by SMART™ PCR. In Gene Cloning and Analysis by RT-PCR (BioTechniques Books, MA), pp. 305-319
SMART™ RACE cDNA Amplification Kit (January 1999) CLONTECHniques XIV (1), 4-6
Zhu, Y.Y.,Machleder, E. M.,Chenchik, A., Li, R. & Siebert, P. M. (2001) Reverse transcriptase template switching: A SMART™ approach for full-length cDNA library construction. BioTechniques 30, 892-897.
Rappolee, D.A., Wang, A., Mark, D., Werb, Z., 1989. Novel method for studying mRNA phenotypes in single or small numbers of cells. J. Cell Biochem. 39, 1.
Cheng, T., Shen, H., Giokas, D., Gere, J., Tenen, D.G., Scadden, D.T., (1996). Temporal mapping of gene expression levels during the differentiation of individual primary hematopoietic cells. Proc. Natl. Acad. Sci. USA. 93, 13158.
O'Brien, D.P., Billadeau, D., Van Ness, B., (1994). RT-PCR assay for detection of transcripts from very few cells using whole cell lysates. Biotechniques 16, 586
Theilgaard-Monch, K, Cowland, J., Borregaard, N. (2001) Profiling of gene expression in individual hematopoietic cells by global mRNA amplification and slot blot analysis. J. Immunol. Methods 252, 175-189
Iscove, N.N., Barbara, M., Gu, M., Gibson, M., Modi, C., & Winegarden, N. (2002) Representation is faithfully preserved in global cDNA amplified exponentially from sub-picogram quantities of mRNA Nat Biotechnol. 20, 940 - 943
Dixon, A., Richardson, K., Lee, P.J., Carter, K., Freeman,T.C. and Freeman, N.P. (1998) Expression profiling of single cells using three prime end amplification (TPEA) PCR Nucl. Acids Res. 26, 4426-4431
Makrigiorgos, G.M., Chakrabarti, S., Zhang, Y., Kaur, M. & Price, B. D. (2002) PCR-based amplification method retaining the quantitative difference between two complex genomes. Nat Biotechnol. 20, 936 - 939
Spirin, KS, Ljubimov, AV, Castellon R, Wiedoeft, O, Marano, M, Sheppard D, Kenney, MC, Brown DJ : (1999) Analysis of gene expression in human bullous keratopathy corneas containing limiting amounts of RNA. Investigative Ophthalmology & Visual Science, 40, 3108-3115
Gonzales P, Zigler, Jr JS, Epstein DL, and Borras T (1999) Identification and isolation of differentially expressed genes from very small tissue samples. Biotechniques 26, 884-892
Vernon, SD, Unger, ER, Rajeevan M, Dimulesco IM, Nisenbaum R and Campbell CE (2000) Reproducibility of alternative probe synthesis approaches for gene expression profiling with arrays. J. Mol. Diag. 2, 124-127
Livesey, FJ, Furukawa, MA, Steffen, MA, Church, GM and Cerko, CL (2000) Microarray analysis of the transcriptional network controlled by the photoreceptor homeobox gene. Crx Curr. Biol. 10, 301-3 10
Zhumabayeva, B., Diatchenko, L., Chenchik, A., and Siebert, P.D. (2001) Use of SMART™-generated cDNA for gene expression studies in multiple human tumours. Biotechniques 30, 158-163
Fink, L, Kohlhoff, S, Stein, MM, Hanze J, Weissmann, N, Rose, F, Akkayagil, E, Manz, D, Grimminger F, Seeger, W and Bohle, RM (2002) cDNA array hybridization after laser-assisted microdissection form nonneoplastic tissue. Am. J. Path. 160 : 81-90
Clark, J.M. (1988). Novel non-templated nucleotide addition reactions catalyzed by prokaryotic and eukaryotic DNA polymerases. Nucleic Acids Res. 16, 9677-9686
Hu, W.S. and Temin, H.M. (1990) Retroviral recombination and reverse transcriptase. Science 250, 1227-1233
Chamberlin and Ryan, in The Enzymes, ed. P. Boyer (Academic Press, New York) pp. 87-108 (1982).
Rosenberg et al., (1987) Gene 56: 125-135
Duguid et al., Proc. Natl. Acad. Sci. USA 85: 5738-5742 (1988)
Sive & St. John, (1988) Nucl. Acids Res. 16, 10937
Chirgwin, J.M., Przybyla, A.E., McDonald, R.J., and Rutter, W.J. et al, (1979) Isolation of biologically active ribonucleic acid from sources enriched in ribonuclease. Biochemistry 18, 5294-5299
Chomczynski, P. & Sacchi, N. (1987). Single-step method of RNA isolation by acid guanidinium thiocyanate-phenol-chloroform extraction. Anal. Biochem. 162, 156-159.
Sambrook, J., Fritsch, E. F. & Maniatis, T. (1989) Molecular Cloning: A Laboratory Manual, Second Edition (Cold Spring Harbor Laboratory, NY)
Farrell, Jr., R. E. (1993) RNA Methodologies-A Lab Guide for Isolation and Characterization (Academic Press, San Diego, CA)
Huang, L, Sitarman K, Gallego, A, Rashtchian, A. (2000) A new highly sensitive two-step RT-PCR system, Focus 22, 6-7
Baugh, L.R., Hill, A.A., Brown, E.L., and Hunter, C.P. (2001) Quantitative analysis of mRNA amplification by in vitro transcription. Nucleic Acids Res. 29, e29
Barnes, W. M. (1994) PCR amplification of up to 35-kb DNA with high fidelity and high yield from bacteriophage templates. Proc. Natl. Acad. Sci. USA 91, 2216-2220.
Kellogg, D. E., Rybalkin, I., Chen, S., Mukhamedova, N., Vlasik, T.,Siebert, P. & Chenchik, A., (1994) TaqStart Antibody: Hotstart PCR facilitated by a neutralizing monoclonal antibody directed against Taq DNA polymerase. BioTechniques 16, 1134-1137.

## Claims

1. A cDNA-RNA hybrid comprising a first strand cDNA synthesis hybridised to RNA wherein the cDNA comprises from the 5'end, an amplifier sequence, 3' to which is an RNA polymerase promoter operably linked to an RNA annealing region, and wherein at least one non-templated nucleotide at the 3' end of the first strand cDNA is hybridised to a template switching oligonucleotide, and wherein the amplifier sequence and the template switching oligonucleotide contain the same sequence.

2. A cDNA-RNA hybrid according to claim 1 wherein the RNA polymerase promoter is a bacteriophage promoter selected from the group consisting of T7, T3 and SP6.

3. A cDNA-RNA hybrid according to any one of the preceding claims wherein the RNA annealing region comprises poly (dT) of about 10 to about 30 T residues in length.

4. A cDNA-RNA hybrid according to any one of the preceding claims wherein the 3' end of the RNA annealing region comprises a VN clamp, wherein V is A, G or C and N is A, G, C or T.

5. A cDNA-mRNA hybrid according to any one of the preceding claims wherein at least one non-templated nucleotide at the 3' end of the first strand cDNA synthesis is deoxycytidine.

6. A cDNA-mRNA hybrid according to any one of the preceding claims wherein at least three non-templated nucleotide at the 3' end of the first strand cDNA synthesis are hybridised to a template switching oligonucleotide.

7. A cDNA-mRNA hybrid according to any one of the preceding claims wherein at least three of the non-templated nucleotides at the 3' end of the first strand cDNA, synthesis are deoxycytidine nucleotides.

8. A cDNA-mRNA hybrid according to any one of the preceding claims wherein the template switching oligonucleotide has at least three guanine residues at its 3' end.

9. A cDNA-mRNA hybrid according to any one of the preceding claims further comprising an amplification primer and wherein, the amplification primer contains the same sequence as the amplifier sequence and the template switching oligonucleotide.

10. A cDNA-mRNA hybrid according to any one of the preceding claims wherein the 3' end of the first strand cDNA synthesis is extended such that it is substantially complementary to the template switching oligonucleotide.

11. A cDNA-mRNA hybrid according to claim 10 wherein the first strand cDNA synthesis is synthesised by a reverse transcriptase, and wherein the reverse transcriptase lacks RNaseH activity but retains wild-type polymerase activity.

12. A method for amplifying RNA in a sample comprising the steps of:
(a) providing a cDNA synthesis oligonucleotide comprising from the 5' end, an amplifier sequence 3' to which is an RNA polymerase promoter operably linked to an RNA annealing region;
(b) annealing the RNA annealing region of the cDNA synthesis oligonucleotide to RNA under suitable conditions to produce a cDNA-RNA complex;
(c) incubating said cDNA-RNA complex under conditions which permit template-dependent extension of the cDNA synthesis oligonucleotide to generate an cDNA-RNA hybrid;
(d) contacting said cDNA-mRNA hybrid with a template switching oligonucleotide under conditions which permit template dependent extension of said cDNA, such that the 3' end of the cDNA comprises a sequence complementary to said template switching oligonucleotide, wherein the amplifier sequence and the template switching oligonucleotide contain the same sequence;
(e) providing an amplification primer under conditions to generate double stranded amplification products corresponding to the first strand cDNA synthesis, such that the cDNA amplification products comprise a double stranded RNA polymerase promoter; and
(f) incubating said cDNA amplification products comprising said double stranded RNA polymerase promoter under conditions that permit *in vitro* transcription to generate amplified RNA.

13. A method according to claim 12 wherein said cDNA-RNA hybrid is incubated with a reverse transcriptase that adds at least one deoxycytidine residue to the 3' end of the first strand cDNA synthesis.

14. A method according to claim 12 or claim 13 wherein at least three non-templated nucleotide at the 3' end of the first strand cDNA synthesis are hybridised to a template switching oligonucleotide.

15. A method according to any one of claims 12 to 14 wherein at least three of the non-templated nucleotides at the 3' end of the first strand cDNA synthesis are deoxycytidine residues.

16. A method according to any one of claims 12 to 15 wherein the reverse transcriptase lacks RNaseH activity but retains wild-type polymerase activity.

17. A method according to any one of claims 12 to 16 wherein said template switching oligonucleotide comprises at least three ribonucleotide residues.

18. A method according to any one of claims 12 to 17 wherein said template switching oligonucleotide comprises at least three guanine residues.

19. A method according to any one of claims 12 to 18 wherein said amplification primer has the same sequence as the amplifier sequence of said cDNA synthesis oligonucleotide.

20. A method according to any one of claims 12 to 19 wherein the double stranded amplification products are obtained by PCR.

21. A method according to any one of claims 12 to 20 wherein the cDNA synthesis oligonucleotide and the PCR primer have the same concentration

22. A method according to any one of claims 12 to 21 wherein the optimum number of cycles to generate the double stranded amplification products is determined by a method comprising the steps of:
(a) providing a plurality of samples with a known amount of RNA;
(b) performing amplification for a defined number of cycles on the plurality of samples;
(c) purifying the double stranded amplification products;
(d) providing for the *in vitro* transcription of the purified amplification products; and
(e) determining the number of amplification cycles that results in the minimum amount of amplified RNA that is required.

23. A method according to any one of claims 12 to 22 wherein the RNA sample is a clinical sample selected from the group consisting of a biopsy, a microdissected tissue, a fine needle aspirate, a flow-sorted cell, a laser captured microdissected cell or a single cell.

24. A method for preparing an expression library of a cell or a cell population comprising the steps of:
(a) providing a cDNA synthesis oligonucleotide comprising from the 5' end, an amplifier sequence, 3' to which is an RNA polymerase promoter operably linked to an RNA annealing region;
(b) contacting said cDNA synthesis oligonucleotide with a population of mRNAs from said cell or cell population under conditions to allow hybridisation of said cDNA synthesis oligonucleotide to mRNA to produce a cDNA-mRNA complex;
(c) incubating said cDNA-mRNA complex under conditions which permit template-dependent extension of said cDNA synthesis oligonucleotide to generate a cDNA-mRNA hybrid;
(d) contacting said cDNA-mRNA hybrid with a template switching oligonucleotide under conditions which permit template dependent extension of said cDNA, such that the 3' end of the cDNA of the cDNA-mRNA hybrid comprises a sequence complementary to said template switching oligonucleotide, wherein the amplifier sequence and the template switching oligonucleotide contain the same sequence;
(e) contacting an amplification primer with said cDNA-mRNA hybrid under conditions that generate double stranded amplification products corresponding to the first strand cDNA synthesis, such that the double stranded cDNA amplification products comprise a double stranded RNA polymerase promoter; and
(f) incubating said double stranded cDNA amplification products comprising said double stranded RNA polymerase promoter under conditions that permit *in vitro* transcription to generate amplified RNA.

25. A method of preparing a cDNA library from a collection of mRNA molecules comprising the steps of:
(a) providing a cDNA synthesis oligonucleotide comprising from the 5' end, an amplifier sequence, 3' to which is an RNA polymerase promoter operably linked to an RNA annealing region;
(b) contacting said cDNA synthesis oligonucleotide with the collection of mRNAs under conditions to allow annealing of said cDNA synthesis oligonucleotide to mRNA produce a cDNA-mRNA complex;
(c) incubating said cDNA-mRNA complex under conditions which permit template-dependent extension of said cDNA synthesis oligonucleotide to generate a cDNA-mRNA hybrid;
(d) contacting said cDNA-mRNA hybrid with a template switching oligonucleotide under conditions which permit template dependent extension of said cDNA of said hybrid, such that the 3' end of the cDNA of the cDNA-mRNA hybrid comprises a sequence complementary to said template switching oligonucleotide, wherein the amplifier sequence and the template switching oligonucleotide contain the same sequence;
(e) contacting a PCR primer with said cDNA-mRNA hybrid under conditions that generate double stranded amplification products corresponding to the first strand cDNA synthesis, such that the double stranded cDNA amplification products comprises a double stranded RNA polymerase, promoter;
(f) incubating said double stranded cDNA amplification products comprising said double stranded RNA polymerase promoter under conditions that permit *in vitro* transcription to generate amplified RNA; and
(g) preparing a cDNA library from the amplified RNA.

26. A method for performing subtractive hybridisation comprising the steps of:
(a) providing a cDNA synthesis oligonucleotide comprising from the 5' end, an amplifier sequence, 3' to which is an RNA polymerase promoter operably linked to an RNA annealing region;
(b) contacting the cDNA synthesis oligonucleotide with a collection of mRNAs under conditions to allow annealing of said cDNA synthesis oligonucleotide to mRNA in said RNA samples to produce a cDNA-mRNA complex;
(c) incubating said cDNA-mRNA hybrid with enzyme, dNTPs and buffer under conditions which permit template-dependent extension of said cDNA synthesis oligonucleotide to generate a cDNA-mRNA hybrid;
(d) contacting said cDNA-mRNA hybrid with a template switching oligonucleotide under conditions which permit template dependent extension of said cDNA of said hybrid, such that the 3' end of the cDNA of the cDNA-mRNA hybrid comprises a sequence complementary to said template switching oligonucleotide, wherein the amplifier sequences and the template switching oligonucleotide contain the same sequence;
(e) contacting an amplification primer with said cDNA-mRNA hybrid under conditions to generate double stranded amplification products corresponding to the first stand cDNA synthesis, such that the double stranded cDNA amplification products comprise a double stranded RNA polymerase promoter;
(f) incubating said double stranded cDNA amplification products comprising said double stranded RNA polymerase promoter under conditions that permit *in vitro* transcription to generate amplified RNA;
(g) contacting said amplified RNA with a single stranded nucleic acid population in the opposite sense to said amplified RNA,
(h) providing for the hybridisation of the sequences present in the amplified RNA and the single stranded nucleic acid population; and
(i) isolating the nucleic acid population that remains single stranded.

27. A method for detecting the expression of a gene of interest comprising the steps of:
(a) providing a cDNA synthesis oligonucleotide comprising from the 5' end, an amplifier sequence, 3' to which is an RNA polymerase promoter operably linked to an RNA annealing region, wherein the RNA annealing region comprises a sequence that is substantially homologous to the mRNA expressed by the gene of interest;
(b) contacting said cDNA synthesis oligonucleotide with a population ofmRNAs in a cell or cell population under conditions to allow annealing of said cDNA synthesis oligonucleotide to mRNA to produce a cDNA-mRNA complex;
(c) incubating said cDNA-mRNA hybrid under conditions which permit template-dependent extension of said cDNA synthesis oligonucleotide to generate a cDNA-mRNA hybrid;
(d) contacting said cDNA-mRNA hybrid with a template switching oligonucleotide under conditions which permit template dependent extension of said cDNA of said hybrid, such that the 3' end of the cDNA of the cDNA-mRNA hybrid comprises a sequence complementary to said template switching oligonucleotide, wherein the amplifier sequence and the template switching oligonucleotide contain the same sequence;
(e) contacting an amplification primer with said cDNA-mRNA hybrid under conditions to generate double stranded amplification products corresponding to the first stand cDNA synthesis, such that the double stranded cDNA amplification products comprise a double stranded RNA polymerase promoter;
(f) incubating said double stranded cDNA amplification products comprising said double stranded RNA polymerase promoter under conditions that permit in *vitro* transcription to generate amplified RNA; and
(g) determining the presence or absence of amplifier RNA, which amplified RNA is complementary to mRNA corresponding to the gene of interest.

28. Use of a cDNA-mRNA hybrid according to any one of claims 1-11 in any one of
the amplification of RNA;
the preparation of a cDNA library;
subtractive hybridisation; or
measuring gene expression.

29. A kit for the amplification of RNA in a sample comprising:
(a) providing a cDNA synthesis oligonucleotide comprising from the 5' end, an amplifier sequence, 3' to which is an RNA polymerase promoter operably linked to an RNA annealing region;
(b) a template switching oligonucleotide that has the same sequence as the amplifier sequence; and
(c) an amplification primer that has the same sequence as the template switching oligonucleotide.

30. The kit according to claim 29, wherein the kit further comprises in a separate container a reverse transcriptase, wherein the reverse transcriptase lacks RNaseH activity but retains wild-type polymerase activity.

31. The kit according to any one of claims 29 to 30, wherein the kit further comprises in a separate container an RNA polymerase specific to the RNA polymerase promoter of the cDNA synthesis oligonucleotide.

32. The kit according to any one of claims claim 29 to 30, wherein the RNA polymerase promoter is selected from a T7, T3 or SP6 RNA polymerase promoter.

33. The kit according to any one of claims 29 to 32, wherein the kit further comprises an amplification buffer and one or more amplification enzymes, wherein the amplification buffer and the amplification enzyme(s) are PCR amplification buffer and PCR amplification enzyme(s).

## Patentansprüche

1. cDNA-RNA-Hybrid, umfassend eine an RNA hybridisierte Erststrang-cDNA-Synthese, wobei die cDNA vom 5'-Ende her eine Verstärkersequenz, an die sich 3' ein in operativer Verknüpfung mit einem RNA-Annealing-Bereich stehender RNA-Polymerase-Promotor anschließt, umfaßt und wobei wenigstens ein ohne Matrize eingebautes Nukleotid am 3'-Ende der Erststrang-cDNA an ein Matrizenwechsel-Oligonukleotid hybridisiert wird und wobei die Verstärkersequenz und das Matrizenwechsel-Oligonukleotid die gleiche Sequenz enthalten.

2. cDNA-RNA-Hybrid nach Anspruch 1, wobei es sich bei dem RNA-Polymerase-Promotor um einen aus der Gruppe T7, T3 und SP6 ausgewählten Bakteriophagenpromotor handelt.

3. cDNA-RNA-Hybrid nach einem der vorhergehenden Ansprüche, wobei der RNA-Annealing-Bereich poly(dT) mit einer Länge von etwa 10 bis etwa 30 Resten umfaßt.

4. cDNA-RNA-Hybrid nach einem der vorhergehenden Ansprüche, wobei das 3'-Ende des RNA-Annealing-Bereichs eine VN-Klammer umfaßt, wobei V für A, G oder C und N für A, G, C oder T steht.

5. cDNA-mRNA-Hybrid nach einem der vorhergehenden Ansprüche, wobei es sich bei wenigstens einem ohne Matrize eingebauten Nukleotid am 3'-Ende der Erststrang-cDNA-Synthese um Desoxycytidin handelt.

6. cDNA-mRNA-Hybrid nach einem der vorhergehenden Ansprüche, wobei wenigstens drei ohne Matrize eingebaute Nukleotide am 3'-Ende der Erststrang-cDNA-Synthese an ein Matrizenwechsel-Oligonukleotid hybridisiert werden.

7. cDNA-mRNA-Hybrid nach einem der vorhergehenden Ansprüche, wobei es sich bei wenigstens drei der ohne Matrize eingebauten Nukleotide am 3'-Ende der Erststrang-cDNA-Synthese um Desoxycytidinnukleotide handelt.

8. cDNA-mRNA-Hybrid nach einem der vorhergehenden Ansprüche, wobei das Matrizenwechsel-Oligonukleotid wenigstens 3 Guaninreste an seinem 3'-Ende aufweist.

9. cDNA-mRNA-Hybrid nach einem der vorhergehenden Ansprüche, ferner umfassend einen Amplifikationsprimer, und wobei der Amplifikationsprimer die gleiche Sequenz wie die Verstärkersequenz und das Matrizenwechsel-Oligonukleotid enthält.

10. cDNA-mRNA-Hybrid nach einem der vorhergehenden Ansprüche, wobei das 3'-Ende der Erststrang-cDNA-Synthese so verlängert wird, daß es weitgehend komplementär zum Matrizenwechsel-Oligonukleotid ist.

11. cDNA-mRNA-Hybrid nach Anspruch 10, wobei die Erststrang-cDNA-Synthese mit einer reversen Transkriptase synthetisiert wird und wobei der reversen Transkriptase RNaseH-Aktivität fehlt, sie jedoch Wildtyp-Polymeraseaktivität behält.

12. Verfahren zur Amplifikation von RNA in einer Probe, bei dem man die folgenden Schritte durchführt:
a) Bereitstellen eines cDNA-Synthese-Oligonukleotids, umfassend vom 5'-Ende her eine Verstärkersequenz, an die sich 3' ein in operativer Verknüpfung mit einem RNA-Annealing-Bereich stehender RNA-Polymerase-Promotor anschließt;
b) Annealing des RNA-Annealing-Bereichs des cDNA-Synthese-Oligonukleotids an RNA unter geeigneten Bedingungen, so daß ein cDNA-RNA-Komplex erhalten wird;
c) Inkubieren des cDNA-RNA-Komplexes unter Bedingungen, die eine matrizenabhängige Verlängerung des cDNA-Synthese-Oligonukleotids gestatten, so daß ein cDNA-RNA-Hybrid erzeugt wird;
d) Inkontaktbringen des cDNA-mRNA-Hybrids mit einem Matrizenwechsel-Oligonukleotid unter Bedingungen, die eine matrizenabhängige Verlängerung der cDNA gestatten, so daß das 3'-Ende der cDNA eine zum Matrizenwechsel-Oligonukleotid komplementäre Sequenz umfaßt, wobei die Verstärkersequenz und das Matrizenwechsel-Oligonukleotid die gleiche Sequenz enthalten;
e) Bereitstellen eines Amplifikationsprimers unter Bedingungen zur Erzeugung von der Erststrang-cDNA-Synthese entsprechenden doppelsträngigen Amplifikationsprodukten, so daß die cDNA-Amplifikationsprodukte einen doppelsträngigen RNA-Polymerase-Promotor umfassen; und
f) Inkubieren der den doppelsträngigen RNA-Polymerase-Promotor umfassenden cDNA-Amplifikationsprodukte unter Bedingungen, die In-vitro-Transkription zur Erzeugung amplifizierter RNA gestatten.

13. Verfahren nach Anspruch 12, wobei das cDNA-RNA-Hybrid mit einer reversen Transkriptase inkubiert wird, die wenigstens einen Desoxycytidinrest an das 3'-Ende der Erststrang-cDNA-Synthese anfügt.

14. Verfahren nach Anspruch 12 oder Anspruch 13, wobei wenigstens drei ohne Matrize eingebaute Nukleotide am 3'-Ende der Erststrang-cDNA-Synthese an ein Matrizenwechsel-Oligonukleotid hybridisiert werden.

15. Verfahren nach einem der Ansprüche 12 bis 14, wobei es sich bei wenigstens drei der ohne Matrize eingebauten Nukleotide am 3'-Ende der Erststrang-cDNA-Synthese um Desoxycytidinnukleotide handelt.

16. Verfahren nach einem der Ansprüche 12 bis 15, wobei der reversen Transkriptase RNaseH-Aktivität fehlt, sie jedoch Wildtyp-Polymeraseaktivität behält.

17. Verfahren nach einem der Ansprüche 12 bis 16, wobei das Matrizenwechsel-Oligonukleotid wenigstens drei Ribonukleotidreste umfaßt.

18. Verfahren nach einem der Ansprüche 12 bis 17, wobei das Matrizenwechsel-Oligonukleotid wenigstens drei Guaninreste umfaßt.

19. Verfahren nach einem der Ansprüche 12 bis 18, wobei der Amplifikationsprimer die gleiche Sequenz wie die Verstärkersequenz des cDNA-Synthese-Oligonukleotids aufweist.

20. Verfahren nach einem der Ansprüche 12 bis 19, wobei die doppelsträngigen Amplifikationsprodukte mittels PCR erhalten werden.

21. Verfahren nach einem der Ansprüche 12 bis 20, wobei das cDNA-Synthese-Oligonukleotid und der PCR-Primer die gleiche Konzentration aufweisen.

22. Verfahren nach einem der Ansprüche 12 bis 21, wobei die optimale Anzahl von Zyklen zur Erzeugung der doppelsträngigen Amplifikationsprodukte mit einem Verfahren bestimmt werden, das die folgenden Schritte umfaßt:
a) Bereitstellen mehrerer Proben mit einer bekannten Menge an RNA;
b) Durchführen der Amplifikation über eine definierte Anzahl von Zyklen mit der Mehrzahl der Proben;
c) Aufreinigen der doppelsträngigen Amplifikationsprodukte;
d) Bereitstellen der aufgereinigten Amplifikationsprodukte für die In-vitro-Transkription und
e) Bestimmen der Anzahl von Amplifikationszyklen, die die Minimalmenge an benötigter amplifizierter RNA ergibt.

23. Verfahren nach einem der Anspüche 12 bis 22, wobei es sich bei der RNA-Probe um eine aus der Gruppe Biopsie, mikrodisseziertes Gewebe, Feinnadelaspirat, Durchfluß-sortierte Zelle, mit Laser eingefangene mikrodissezierte Zelle oder Einzelzelle ausgewählte klinische Probe handelt.

24. Verfahren zur Herstellung einer Expressionsbibliothek einer Zelle oder Zellpopulation, bei dem man die folgenden Schritte durchführt:
a) Bereitstellen eines cDNA-Synthese-Oligonukleotids, umfassend vom 5'-Ende her eine Verstärkersequenz, an die sich 3' ein in operativer Verknüpfung mit einem RNA-Annealing-Bereich stehender RNA-Polymerase-Promotor anschließt;
b) Inkontaktbringen des cDNA-Synthese-Oligonukleotids mit einer Population von mRNAs aus der Zelle oder Zellpopulation unter Bedingungen, die die Hybridisierung des cDNA-Synthese-Oligonukleotids an mRNA unter Erhalt eines cDNA-mRNA-Komplexes gestatten;
c) Inkubieren des cDNA-mRNA-Komplexes unter Bedingungen, die eine matrizenabhängige Verlängerung des cDNA-Synthese-Oligonukleotids gestatten, so daß ein cDNA-mRNA-Hybrid erzeugt wird;
d) Inkontaktbringen des cDNA-mRNA-Hybrids mit einem Matrizenwechsel-Oligonukleotid unter Bedingungen, die eine matrizenabhängige Verlängerung der cDNA gestatten, so daß das 3'-Ende der cDNA des cDNA-mRNA-Hybrids eine zum Matrizenwechsel-Oligonukleotid komplementäre Sequenz umfaßt, wobei die Verstärkersequenz und das Matrizenwechsel-Oligonukleotid die gleiche Sequenz enthalten;
e) Inkontaktbringen eines Amplifikationsprimers mit dem cDNA-mRNA-Hybrid unter Bedingungen, bei denen der Erststrang-cDNA-Synthese entsprechende doppelsträngige Amplifikationsprodukte erzeugt werden, so daß die doppelsträngigen cDNA-Amplifikationsprodukte einen doppelsträngigen RNA-Polymerase-Promotor umfassen; und
f) Inkubieren der den doppelsträngigen RNA-Polymerase-Promotor umfassenden doppelsträngigen cDNA-Amplifikationsprodukte unter Bedingungen, die In-vitro-Transkription zur Erzeugung amplifizierter RNA gestatten.

25. Verfahren zur Herstellung einer cDNA-Bibliothek aus einer Sammlung von mRNA-Molekülen, bei dem man die folgenden Schritte durchführt:
a) Bereitstellen eines cDNA-Synthese-Oligonukleotids, umfassend vom 5'-Ende her eine Verstärkersequenz, an die sich 3' ein in operativer Verknüpfung mit einem RNA-Annealing-Bereich stehender RNA-Polymerase-Promotor anschließt;
b) Inkontaktbringen des cDNA-Synthese-Oligonukleotids mit der Sammlung von mRNAs unter Bedingungen, bei denen durch Annealing des cDNA-Synthese-Oligonukleotids an mRNA der Erhalt eines cDNA-mRNA-Komplexes gestattet wird;
c) Inkubieren des cDNA-mRNA-Komplexes unter Bedingungen, die eine matrizenabhängige Verlängerung des cDNA-Synthese-Oligonukleotids gestatten, so daß ein cDNA-mRNA-Hybrid erzeugt wird;
d) Inkontaktbringen des cDNA-mRNA-Hybrids mit einem Matrizenwechsel-Oligonukleotid unter Bedingungen, die eine matrizenabhängige Verlängerung der cDNA des Hybrids gestatten, so daß das 3'-Ende der cDNA des cDNA-mRNA-Hybrids eine zum Matrizenwechsel-Oligonukleotid komplementäre Sequenz umfaßt, wobei die Verstärkersequenz und das Matrizenwechsel-Oligonukleotid die gleiche Sequenz enthalten;
e) Inkontaktbringen eines PCR-Primers mit dem cDNA-mRNA-Hybrid unter Bedingungen, bei denen der Erststrang-cDNA-Synthese entsprechende doppelsträngige Amplifikationsprodukte erzeugt werden, so daß die doppelsträngigen cDNA-Amplifikationsprodukte einen doppelsträngigen RNA-Polymerase-Promotor umfassen;
f) Inkubieren der den doppelsträngigen RNA-Polymerase-Promotor umfassenden doppelsträngigen cDNA-Amplifikationsprodukte unter Bedingungen, die In-vitro-Transkription zur Erzeugung amplifizierter RNA gestatten; und
g) Herstellen einer cDNA-Bibliothek aus der amplifizierten RNA.

26. Verfahren zur Durchführung von subtraktiver Hybridisierung, bei dem man die folgenden Schritte durchführt:
a) Bereitstellen eines cDNA-Synthese-Oligonukleotids, umfassend vom 5'-Ende her eine Verstärkersequenz, an die sich 3' ein in operativer Verknüpfung mit einem RNA-Annealing-Bereich stehender RNA-Polymerase-Promotor anschließt;
b) Inkontaktbringen des cDNA-Synthese-Oligonukleotids mit einer Sammlung von mRNAs unter Bedingungen, bei denen durch Annealing des cDNA-Synthese-Oligonukleotids an mRNA in der RNA-Probe der Erhalt eines cDNA-mRNA-Komplexes gestattet wird;
c) Inkubieren des cDNA-mRNA-Hybrids mit Enzym, dNTPs und Puffer unter Bedingungen, die eine matrizenabhängige Verlängerung des cDNA-Synthese-Oligonukleotids gestatten, so daß ein cDNA-mRNA-Hybrid erzeugt wird;
d) Inkontaktbringen des cDNA-mRNA-Hybrids mit einem Matrizenwechsel-Oligonukleotid unter Bedingungen, die eine matrizenabhängige Verlängerung der cDNA des Hybrids gestatten, so daß das 3'-Ende der cDNA des cDNA-mRNA-Hybrids eine zum Matrizenwechsel-Oligonukleotid komplementäre Sequenz umfaßt, wobei die Verstärkersequenz und das Matrizenwechsel-Oligonukleotid die gleiche Sequenz enthalten;
e) Inkontaktbringen eines Amplifikationsprimers mit dem cDNA-mRNA-Hybrid unter Bedingungen, bei denen der Erststrang-cDNA-Synthese entsprechende doppelsträngige Amplifikationsprodukte erzeugt werden, so daß die doppelsträngigen cDNA-Amplifikationsprodukte einen doppelsträngigen RNA-Polymerase-Promotor umfassen;
f) Inkubieren der den doppelsträngigen RNA-Polymerase-Promotor umfassenden doppelsträngigen cDNA-Amplifikationsprodukte unter Bedingungen, die In-vitro-Transkription zur Erzeugung amplifizierter RNA gestatten;
g) Inkontaktbringen der amplifizierten RNA mit einer einzelsträngigen Nukleinsäurepopulation im entgegengesetzten Sinn zur amplifizierten RNA;
h) Vorsehen der Hybridisierung der in der amplifizierten RNA vorhandenen Sequenzen und der einzelsträngigen Nukleinsäurepopulation; und
i) Isolieren der der verbliebenen einzelsträngigen Nukleinsäurepopulation.

27. Verfahren zum Nachweis der Expression eines interessierenden Gens, wobei man die folgenden Schritte durchführt:
a) Bereitstellen eines cDNA-Synthese-Oligonukleotids, umfassend vom 5'-Ende her eine Verstärkersequenz, an die sich 3' ein in operativer Verknüpfung mit einem RNA-Annealing-Bereich stehender RNA-Polymerase-Promotor anschließt, wobei der RNA-Annealing-Bereich eine Sequenz umfaßt, die zu der von dem interessierenden Gen exprimierten mRNA weitgehend homolog ist;
b) Inkontaktbringen des cDNA-Synthese-Oligonukleotids mit einer Population von mRNAs in einer Zelle oder Zellpopulation unter Bedingungen, bei denen durch Annealing des cDNA-Synthese-Oligonukleotids an mRNA der Erhalt eines cDNA-mRNA-Komplexes gestattet wird;
c) Inkubieren des cDNA-mRNA-Hybrids unter Bedingungen, die eine matrizenabhängige Verlängerung des cDNA-Synthese-Oligonukleotids gestatten, so daß ein cDNA-mRNA-Hybrid erzeugt wird;
d) Inkontaktbringen des cDNA-mRNA-Hybrids mit einem Matrizenwechsel-Oligonukleotid unter Bedingungen, die eine matrizenabhängige Verlängerung der cDNA des Hybrids gestatten, so daß das 3'-Ende der cDNA des cDNA-mRNA-Hybrids eine zum Matrizenwechsel-Oligonukleotid komplementäre Sequenz umfaßt, wobei die Verstärkersequenz und das Matrizenwechsel-Oligonukleotid die gleiche Sequenz enthalten;
e) Inkontaktbringen eines Amplifikationsprimers mit dem cDNA-mRNA-Hybrid unter Bedingungen, bei denen der Erststrang-cDNA-Synthese entsprechende doppelsträngige Amplifikationsprodukte erzeugt werden, so daß die doppelsträngigen cDNA-Amplifikationsprodukte einen doppelsträngigen RNA-Polymerase-Promotor umfassen;
f) Inkubieren der den doppelsträngigen RNA-Polymerase-Promotor umfassenden doppelsträngigen cDNA-Amplifikationsprodukte unter Bedingungen, die In-vitro-Transkription zur Erzeugung amplifizierter RNA gestatten; und
g) Bestimmen des Vorhandenseins oder der Abwesenheit amplifizierter RNA, wobei die amplifizierte RNA zu dem interessierenden Gen entsprechender mRNA komplementär ist.

28. Verwendung eines cDNA-mRNA-Hybrids nach einem der Ansprüche 1-11 in einem der folgenden Verfahren:
der Amplifikation von RNA;
der Herstellung einer cDNA-Bibliothek;
subtraktiver Hybridisierung; oder
Messen der Genexpression.

29. Kit zur Amplifikation von RNA in einer Probe, umfassend:
a) Bereitstellen eines cDNA-Synthese-Oligonukleotids, umfassend vom 5'-Ende her eine Verstärkersequenz, an die sich 3' ein in operativer Verknüpfung mit einem RNA-Annealing-Bereich stehender RNA-Polymerase-Promotor anschließt;
b) ein Matrizenwechsel-Oligonukleotid, das die gleiche Sequenz wie die Verstärkersequenz aufweist; und
c) einen Amplifikationsprimer, der die gleiche Sequenz wie das Matrizenwechsel-Oligonukleotid aufweist.

30. Kit nach Anspruch 29, wobei der Kit ferner in einem getrennten Behälter eine reverse Transkriptase umfaßt, wobei der reversen Transkriptase RNaseH-Aktivität fehlt, sie jedoch Wildtyp-Polymeraseaktivität behält.

31. Kit nach einem der Ansprüche 29 bis 30, wobei der Kit ferner in einem getrennten Behälter eine für den RNA-Polymerase-Promotor des cDNA-Synthese-Oligonukleotids spezifische RNA-Polymerase umfaßt.

32. Kit nach einem der Ansprüche 29 bis 30, wobei der RNA-Polymerase-Promotor aus einem T7-, T3- oder SP6-RNA-Polymerase-Promotor ausgewählt ist.

33. Kit nach einem der Ansprüche 29 bis 32, wobei der Kit ferner einen Amplifikationspuffer sowie ein oder mehr Amplifikationsenzyme umfaßt, wobei es sich bei dem Amplifikationspuffer bzw. dem (den) Amplifikationsenzym(en) um PCR-Amplifikationspuffer bzw. PCR-Amplifikationsenzym(e) handelt.

## Revendications

1. Hybride d'ADNc-ARN comprenant la synthèse d'un premier brin d' ADNc hybridé à un ARN, dans lequel l'ADNc comprend, à partir de l'extrémité 5', une séquence amplificatrice à partir de laquelle il y a en 3' un promoteur d'ARN polymérase lié, de manière opérationnelle, à une région d'annelage de l'ARN et dans lequel au moins un nucléotide non matrice, à l'extrémité 3' du premier brin de l'ADNc, est hybridé avec un oligonucléotide de changement de matrice et dans lequel la séquence amplificatrice et l'oligonucléotide de changement de matrice contiennent la même séquence.

2. Hybride d'ADNc-ARN selon la revendication 1, dans lequel le promoteur de l'ARN polymérase est un promoteur de bactériophage choisi dans le groupe composé des T7, T3 et SP6.

3. Hybride d'ADNc-ARN selon l'une quelconque des revendications précédentes, dans lequel la région d'annelage de l'ARN comprend un poly(dT) d'une longueur d'environ 10 à environ 30 résidus T.

4. Hybride d'ADNc-ARN selon l'une quelconque des revendications précédentes, dans lequel l'extrémité 3' de la région d'annelage de l'ARN comprend une attache VN, dans laquelle le V est un A, un G ou un C et le N est un A, un G, un C ou un T.

5. Hybride d'ADNc-ARNm selon l'une quelconque des revendications précédentes, dans lequel au moins un nucléotide non matrice à l'extrémité 3' de la synthèse du premier brin de l'ADNc est une désoxycytidine.

6. Hybride d'ADNc-ARNm selon l'une quelconque des revendications précédentes, dans lequel au moins trois nucléotides non matrices à l'extrémité 3' de la synthèse du premier brin de l'ADNc sont hybridés avec un oligonucléotide de changement de matrice.

7. Hybride d'ADNc-ARNm selon l'une quelconque des revendications précédentes, dans lequel au moins trois nucléotides non matrices à l'extrémité 3' de la synthèse du premier brin de l'ADNc sont des nucléotides de désoxycytidine.

8. Hybride d'ADNc-ARNm selon l'une quelconque des revendications précédentes, dans lequel l'oligonucléotide de changement de matrice a au moins trois résidus de guanine au niveau de son extrémité 3'.

9. Hybride d'ADNc-ARNm, selon l'une quelconque des revendications précédentes, comprenant en outre une amorce d'amplification et dans lequel l'amorce d'amplification contient la même séquence que la séquence amplificatrice et l'oligonucléotide de changement de matrice.

10. Hybride d'ADNc-ARNm selon l'une quelconque des revendications précédentes, dans lequel l'extrémité 3' de la synthèse du premier brin de l'ADNc est allongée de sorte qu'elle soit substantiellement complémentaire de l'oligonucléotide de changement de matrice.

11. Hybride d'ADNc-ARNm selon la revendication 10, dans lequel la synthèse du premier brin de l'ADNc est synthétisée par une transcriptase inverse et dans lequel la transcriptase inverse est dépourvue d'activité de RNAseH mais conserve une activité de polymérase de type sauvage.

12. Procédé d'amplification d'ARN dans un échantillon comprenant les étapes consistant à :
(a) fournir un oligonucléotide de synthèse d'ADNc comprenant, à partir de l'extrémité 5', une séquence amplificatrice à partir de laquelle il y a en 3' un promoteur d'ARN polymérase lié, de manière opérationnelle, à une région d'annelage de l'ARN ;
(b) anneler la région d'annelage de l'ARN de l'oligonucléotide de synthèse d'ADNc avec un ARN dans des conditions appropriées pour produire un complexe d'ADNc-ARN ;
(c) incuber ledit complexe d'ADNc-ARN dans des conditions qui permettent un allongement, dépendant d'une matrice, de l'oligonucléotide de synthèse d'ADNc pour générer un hybride d'ADNc-ARN ;
(d) mettre en contact ledit hybride d'ADNc-ARNm avec un oligonucléotide de changement de matrice dans des conditions qui permettent un allongement, dépendant d'une matrice, dudit ADNc de sorte que l'extrémité 3' de l'ADNc comprenne une séquence complémentaire dudit oligonucléotide de changement de matrice, dans lequel la séquence amplificatrice et l'oligonucléotide de changement de matrice contiennent la même séquence ;
(e) fournir une amorce d'amplification dans des conditions permettant de générer des produits d'amplification en double brin correspondant à la synthèse du premier brin de l'ADNc, de sorte que les produits d'amplification de l'ADNc comprennent un promoteur d'ARN polymérase en double brin ; et
(f) incuber lesdits produits d'amplification de l'ADNc, comprenant ledit promoteur d'ARN polymérase en double brin, dans des conditions qui permettent une transcription *in vitro* pour générer de l'ARN amplifié.

13. Procédé selon la revendication 12, dans lequel ledit hybride d'ADNc-ARN est incubé avec une transcriptase inverse qui ajoute au moins un résidu de désoxycytidine à l'extrémité 3' de la synthèse du premier brin de l'ADNc.

14. Procédé selon la revendication 12 ou la revendication 13, dans lequel au moins trois nucléotides non matrices à l'extrémité 3' de la synthèse du premier brin de l'ADNc sont hybridés avec un oligonucléotide de changement de matrice.

15. Procédé selon l'une quelconque des revendications 12 à 14, dans lequel au moins trois des nucléotides non matrices à l'extrémité 3' de la synthèse du premier brin de l'ADNc sont des résidus de désoxycytidine.

16. Procédé selon l'une quelconque des revendications 12 à 15, dans lequel la transcriptase inverse est dépourvue d'activité de RNAseH mais conserve une activité de polymérase de type sauvage.

17. Procédé selon l'une quelconque des revendications 12 à 16, dans lequel ledit oligonucléotide de changement de matrice comprend au moins trois résidus ribonucléotidiques.

18. Procédé selon l'une quelconque des revendications 12 à 17, dans lequel ledit oligonucléotide de changement de matrice comprend au moins trois résidus de guanine.

19. Procédé selon l'une quelconque des revendications 12 à 18, dans lequel ladite amorce d'amplification a la même séquence que la séquence amplificatrice dudit oligonucléotide de synthèse de l'ADNc.

20. Procédé selon l'une quelconque des revendications 12 à 19, dans lequel les produits d'amplification en double brin sont obtenus par ACP.

21. Procédé selon l'une quelconque des revendications 12 à 20, dans lequel l'oligonucléotide de synthèse de l'ADNc et l'amorce d'ACP ont la même concentration.

22. Procédé selon l'une quelconque des revendications 12 à 21, dans lequel le nombre optimum de cycles pour générer les produits d'amplification en double brin est déterminé par un procédé comprenant les étapes consistant à :
(a) fournir une multiplicité d'échantillons ayant une quantité connue d'ARN ;
(b) exécuter une amplification, pour un nombre défini de cycles, sur la multiplicité d'échantillons ;
(c) purifier les produits d'amplification en double brin ;
(d) pourvoir une transcription *in vitro* des produits d'amplification purifiés ; et
(e) déterminer le nombre de cycles d'amplification qui conduit à la quantité minimale requise d'ARN amplifié.

23. Procédé selon l'une quelconque des revendications 12 à 22, dans lequel l'échantillon d'ARN est un échantillon clinique choisi dans le groupe composé d'une biopsie, d'un tissu microdisséqué, d'un produit d'aspiration d'une aiguille fine, d'une cellule triée en flux, d'une cellule microdisséquée capturée au laser ou d'une seule cellule.

24. Procédé de préparation d'une bibliothèque d'expression d'une cellule ou d'une population de cellules comprenant les étapes consistant à :
(a) fournir un oligonucléotide de synthèse d'ADNc comprenant, à partir de l'extrémité 5', une séquence amplificatrice à partir de laquelle il y a en 3' un promoteur d'ARN polymérase lié, de manière opérationnelle, à une région d'annelage de l'ARN ;
(b) mettre en contact ledit oligonucléotide de synthèse d'ADNc avec une population d'ARNm provenant de ladite cellule ou de ladite population de cellules dans des conditions qui permettent une hybridation dudit oligonucléotide de synthèse d'ADNc avec l'ARNm pour produire un complexe d'ADNc-ARNm ;
(c) incuber ledit complexe d'ADNc-ARNm dans des conditions qui permettent un allongement, dépendant d'une matrice, dudit oligonucléotide de synthèse d'ADNc pour générer un hybride d'ADNc-ARNm ;
(d) mettre en contact ledit hybride d'ADNc-ARNm avec un oligonucléotide de changement de matrice dans des conditions qui permettent un allongement, dépendant d'une matrice, dudit ADNc de sorte que l'extrémité 3' de l'ADNc de l'hybride d'ADNc-ARNm comprenne une séquence complémentaire dudit oligonucléotide de changement de matrice, dans lequel la séquence amplificatrice et l'oligonucléotide de changement de matrice contiennent la même séquence ;
(e) mettre en contact une amorce d'amplification avec ledit hybride d'ADNc-ARNm dans des conditions qui permettent de générer des produits d'amplification en double brin correspondant à la synthèse du premier brin de l'ADNc, de sorte que les produits d'amplification de l'ADNc en double brin comprennent un promoteur d'ARN polymérase en double brin ; et
(f) incuber lesdits produits d'amplification de l'ADNc en double brin, comprenant ledit promoteur d'ARN polymérase en double brin, dans des conditions qui permettent une transcription *in vitro* pour générer de l'ARN amplifié.

25. Procédé de préparation d'une bibliothèque d'ADNc à partir d'une collection d'ARNm, comprenant les étapes consistant à :
(a) fournir un oligonucléotide de synthèse d'ADNc comprenant, à partir de l'extrémité 5', une séquence amplificatrice à partir de laquelle il y a en 3' un promoteur d'ARN polymérase lié, de manière opérationnelle, à une région d'annelage de l'ARN ;
(b) mettre en contact ledit oligonucléotide de synthèse d'ADNc avec la collection d'ARNm dans des conditions permettant d'anneler ledit oligonucléotide de synthèse de l'ADNc à un ARNm pour produire un complexe d'ADNc-ARNm ;
(c) incuber ledit complexe d'ADNc-ARNm dans des conditions qui permettent un allongement, dépendant d'une matrice, dudit oligonucléotide de synthèse de l'ADNc pour générer un hybride d'ADNc-ARNm ;
(d) mettre en contact ledit hybride d'ADNc-ARNm avec un oligonucléotide de changement de matrice dans des conditions qui permettent un allongement, dépendant d'une matrice, dudit ADNc dudit hybride, de sorte que l'extrémité 3' de l'ADNc de l'hybride d'ADNc-ARNm comprenne une séquence complémentaire dudit oligonucléotide de changement de matrice, dans lequel la séquence amplificatrice et l'oligonucléotide de changement de matrice contiennent la même séquence ;
(e) mettre en contact une amorce d'ACP avec ledit hybride d'ADNc-ARNm dans des conditions permettant de générer des produits d'amplification en double brin correspondant à la synthèse du premier brin de l'ADNc, de sorte que les produits d'amplification de l'ADNc en double brin comprennent un promoteur d'ARN polymérase en double brin ;
(f) incuber lesdits produits d'amplification de l'ADNc en double brin, comprenant ledit promoteur d'ARN polymérase en double brin, dans des conditions qui permettent une transcription *in vitro* pour générer de l'ARN amplifié ; et
(g) préparer une bibliothèque d'ADNc à partir de l'ARN amplifié.

26. Procédé d'exécution d'une hybridation soustractive comprenant les étapes consistant à :
(a) fournir un oligonucléotide de synthèse d'ADNc comprenant, à partir de l'extrémité 5', une séquence amplificatrice à partir de laquelle il y a en 3' un promoteur d'ARN polymérase lié, de manière opérationnelle, à une région d'annelage de l'ARN ;
(b) mettre en contact ledit oligonucléotide de synthèse d'ADNc avec une collection d'ARNm dans des conditions permettant d'anneler ledit oligonucléotide de synthèse de l'ADNc avec un ARNm dans ledit échantillon d'ARN pour produire un complexe d'ADNc-ARNm ;
(c) incuber ledit hybride d'ADNc-ARNm avec une enzyme, des dNTP et un tampon, dans des conditions qui permettent un allongement, dépendant d'une matrice, dudit oligonucléotide de synthèse de l'ADNc pour générer un hybride d'ADNc-ARNm ;
(d) mettre en contact ledit hybride d'ADNc-ARNm avec un oligonucléotide de changement de matrice dans des conditions qui permettent un allongement, dépendant d'une matrice, dudit ADNc dudit hybride, de sorte que l'extrémité 3' de l'ADNc de l'hybride d'ADNc-ARNm comprenne une séquence complémentaire dudit oligonucléotide de changement de matrice, dans lequel la séquence amplificatrice et l'oligonucléotide de changement de matrice contiennent la même séquence ;
(e) mettre en contact une amorce d'amplification avec ledit hybride d'ADNc-ARNm dans des conditions qui permettent de générer des produits d'amplification en double brin correspondant à la synthèse du premier brin de l'ADNc, de sorte que les produits d'amplification de l'ADNc en double brin comprennent un promoteur d'ARN polymérase en double brin ;
(f) incuber lesdits produits d'amplification de l'ADNc en double brin, comprenant ledit promoteur d'ARN polymérase en double brin, dans des conditions qui permettent une transcription *in vitro* pour générer de l'ARN amplifié ;
(g) mettre en contact ledit ARN amplifié avec une population d'acides nucléiques en simple brin, dans le sens opposé audit ARN amplifié ;
(h) pourvoir l'hybridation des séquences présentes dans l'ARN amplifié et la population d'acides nucléiques en simple brin ; et
(i) isoler la population d'acides nucléiques qui reste en simple brin.

27. Procédé de détection de l'expression d'un gène d'intérêt comprenant les étapes consistant à :
(a) fournir un oligonucléotide de synthèse d'ADNc comprenant, à partir de l'extrémité 5', une séquence amplificatrice à partir de laquelle il y a en 3' un promoteur d'ARN polymérase lié, de manière opérationnelle, à une région d'annelage de l'ARN, dans lequel la région d'annelage de l'ARN comprend une séquence qui est substantiellement homologue à l'ARNm exprimé par le gène d'intérêt ;
(b) mettre en contact ledit oligonucléotide de synthèse d'ADNc avec une population d'ARNm dans une cellule ou une population de cellules dans des conditions permettant d'anneler ledit oligonucléotide de synthèse de l'ADNc à un ARNm pour produire un complexe d'ADNc-ARNm ;
(c) incuber ledit hybride d'ADNc-ARNm dans des conditions qui permettent un allongement, dépendant d'une matrice, dudit oligonucléotide de synthèse de l'ADNc pour générer un hybride d'ADNc-ARNm ;
(d) mettre en contact ledit hybride d'ADNc-ARNm avec un oligonucléotide de changement de matrice dans des conditions qui permettent un allongement, dépendant d'une matrice, dudit ADNc dudit hybride, de sorte que l'extrémité 3' de l'ADNc de l'hybride d'ADNc-ARNm comprenne une séquence complémentaire dudit oligonucléotide de changement de matrice, dans lequel la séquence amplificatrice et l'oligonucléotide de changement de matrice contiennent la même séquence ;
(e) mettre en contact une amorce d'amplification avec ledit hybride d'ADNc-ARNm dans des conditions qui permettent de générer des produits d'amplification en double brin correspondant à la synthèse du premier brin de l'ADNc, de sorte que les produits d'amplification de l'ADNc en double brin comprennent un promoteur d'ARN polymérase en double brin ;
(f) incuber lesdits produits d'amplification de l'ADNc en double brin, comprenant ledit promoteur d'ARN polymérase en double brin, dans des conditions qui permettent une transcription *in vitro* pour générer de l'ARN amplifié ; et
(g) déterminer la présence ou l'absence d'ARN amplifié, lequel ARN amplifié est complémentaire de l'ARNm correspondant au gène d'intérêt.

28. Utilisation d'un hybride d'ADNc-ARNm, selon l'une quelconque des revendications 1 à 11, dans n'importe quelle étape parmi :
l'amplification d'un ARN ;
la préparation d'une bibliothèque d'ADNc ;
une hybridation soustractive ; ou
une mesure de l'expression d'un gène.

29. Kit pour l'amplification d'ARN dans un échantillon comprenant les étapes consistant à :
(a) fournir un oligonucléotide de synthèse d'ADNc comprenant, à partir de l'extrémité 5', une séquence amplificatrice à partir de laquelle il y a en 3' un promoteur d'ARN polymérase lié, de manière opérationnelle, à une région d'annelage de l'ARN ;
(b) un oligonucléotide de changement de matrice qui a la même séquence que la séquence amplificatrice ; et
(c) une amorce d'amplification qui a la même séquence que l'oligonucléotide de changement de matrice.

30. Kit selon la revendication 29, dans lequel le kit comprend en outre, dans un conteneur séparé, une transcriptase inverse, dans lequel la transcriptase inverse est dépourvue d'activité de RNAseH mais conserve une activité de polymérase de type sauvage.

31. Kit selon l'une quelconque des revendications 29 à 30, dans lequel le kit comprend en outre, dans un conteneur séparé, une ARN polymérase spécifique du promoteur d'ARN polymérase de l'oligonucléotide de synthèse de l'ADNc.

32. Kit selon l'une quelconque des revendications 29 à 30, dans lequel le promoteur de l'ARN polymérase est choisi parmi le promoteur de l'ARN polymérase d'un T7, d'un T3 ou d'un SP6.

33. Kit selon l'une quelconque des revendications 29 à 32, dans lequel le kit comprend en outre un tampon d'amplification et une ou plusieurs enzyme(s) d'amplification, dans lequel le tampon d'amplification et la (les) enzyme(s) d'amplification sont un tampon d'amplification pour ACP et une (des) enzyme(s) d'amplification pour ACP.
